# EUROPEAN PATENT APPLICATION

(11) **EP 4 403 646 A2**
(43) Date of publication of application: **24.07.2024**
(21) Application number: 24165571.1
(22) Date of filing: 13.07.2018
(51) Int. Cl.: C12Q 1/6827

(54) **DNA TARGETS AS TISSUE-SPECIFIC METHYLATION MARKERS**

(30) Priority: 13.07.2017 US 201762531988 P
(62) Divisional of application: 18756502.3
(71) Applicant: Yissum Research and Development Company of the Hebrew University of Jerusalem Ltd., 9139002 Jerusalem (IL); Hadasit Medical Research Services and Development Ltd., 9112001 Jerusalem (IL)
(72) Inventor: DOR, Yuval, 9774415 Jerusalem (IL); SHEMER, Ruth, 9078910 Mevasseret Zion (IL); GLASER, Benjamin, 9692852 Jerusalem (IL); MAGENHEIM, Judith, 9045410 Efrat (IL); NEIMAN, Daniel, 7936700 Doar-Na Lachish Darom (IL); WERMAN, Roni, 9358523 Jerusalem (IL); ZEMMOUR, Hai, 9622428 Jerusalem (IL); MOSS, Joshua, 9319004 Jerusalem (IL); FOX, Ilana, 9234803 Jerusalem (IL); PIYANZIN, Sheina, 9678808 Jerusalem (IL)
(74) Representative: Boult Wade Tennant LLP

(57) **Abstract**

A method of ascertaining the methylation status of a double-stranded, cell-free DNA molecule in a specimen is disclosed. The method comprises ascertaining the methylation status of at least two methylation sites of the same double-stranded cell-free DNA molecule, wherein said double-stranded, cell-free DNA molecule comprises a nucleotide sequence which comprises no more than 300 base pairs and is comprised in a sequence as set forth in any one of SEQ ID NOs: 2-117 or 121-177.

## Description

### FIELD AND BACKGROUND OF THE INVENTION

The present invention contemplates novel target sequences that can be used as tissue-specific methylation markers.

It has been known for decades that plasma contains small fragments of cell-free circulating DNA (cfDNA) derived from dead cells (on average 1000 genome equivalents per ml). While the mechanisms underlying the release and clearance of cfDNA remain obscure, the phenomenon is rapidly being exploited for a variety of applications with clinical relevance. The recognition that fragments of fetal DNA travel briefly in maternal circulation has opened the way for next generation sequencing (NGS)-based prenatal testing to identify fetal trisomies and other genetic aberrations, potentially replacing amniocentesis. In cancer biology, tumors are known to release DNA (including tumor-specific somatic mutations) into the circulation, providing means for liquid biopsies to monitor tumor dynamics and genomic evolution. In addition, cfDNA has been used to detect graft cell death after kidney, liver or heart transplantation, based on single nucleotide polymorphisms (SNPs) distinguishing the DNA of donor from that of recipients. In all these cases, genetic differences exist between the DNA sequence of the tissue of interest (fetus, tumor or graft) and that of the host, providing the basis for highly specific assays.

Blood levels of cfDNA are known to increase under multiple additional conditions such as traumatic brain injury, cardiovascular disease, sepsis and intensive exercise. However in these cases, the source of elevated cfDNA is unknown, greatly compromising the utility of cfDNA as a diagnostic or prognostic tool. For example, cfDNA could originate from parenchymal cells of the injured tissue, but also from dying inflammatory cells.

Despite having an identical nucleotide sequence, the DNA of each cell type in the body carries unique epigenetic marks correlating with its gene expression profile. In particular, DNA methylation, serving to repress nontranscribed genes, is a fundamental aspect of tissue identity. Methylation patterns are unique to each cell type, conserved among cells of the same type in the same individual and between individuals, and are highly stable under physiologic or pathologic conditions. Therefore, it may be possible to use the DNA methylation pattern of cfDNA to determine its tissue of origin and hence to infer cell death in the source organ.

Theoretically, such an approach could identify the rate of cell death in a tissue of interest, taking into account the total amount of cfDNA, the fraction derived from a tissue of interest, and the estimated half-life of cfDNA (15-120 minutes). Note that since the approach relies on normal, stable markers of cell identity, it cannot identify the nature of the pathology (e.g. distinguishing cfDNA derived from dead tumor cells or dead wild type cells due to trauma or inflammation in the same tissue). The potential uses of a highly sensitive, minimally invasive assay of tissue specific cell death include early, precise diagnosis as well as monitoring response to therapy in both a clinical and drug-development setting.

A classic example of tissue-specific DNA methylation is provided by the insulin gene promoter, which is unmethylated in insulin-producing pancreatic β-cells and methylated elsewhere. Recent studies have identified unmethylated insulin promoter DNA in the circulation of newly diagnosed T1D patients as well as in islet graft recipients, likely reflecting both autoimmune and alloimmune destruction of β -cells (Akirav E.M.et al. Proceedings of the National Academy of Sciences of the United States of America, 108, 19018-19023 (2011); Lebastchi J et al., Diabetes 62, 1676-1680 (2013); Husseiny M. I. Plos one 9 e94591 (2014; and Herold K.C. et al., J Clin Invest. Doi:10.1172/jc178142 (2015)).

Additional background art includes Bidshahri et al., The Journal of Molecular Diagnostics, Vol. 18, No. 2, March 2016, Usmani-Brown et al., Endocrinology 155: 3694-3698, 2014; International PCT Publication No. WO2013131083, WO 2014138133, WO201101728, WO2015/159292 and WO2015169947.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### SUMMARY OF THE INVENTION

According to an aspect of some embodiments of the present invention there is provided a method of ascertaining the methylation status of a double-stranded, cell-free DNA molecule in a specimen, the method comprising ascertaining the methylation status of at least two methylation sites of the same double-stranded cell-free DNA molecule, wherein the double-stranded, cell-free DNA molecule comprises a nucleotide sequence which comprises no more than 300 base pairs and is comprised in a sequence as set forth in any one of SEQ ID NOs: 2-117 or 121-177, thereby ascertaining the methylation status of a double-stranded, cell-free DNA molecule.

According to an aspect of some embodiments of the present invention there is provided a method of detecting death of a cell type or tissue in a subject comprising determining whether a double-stranded, cell-free DNA molecule comprised in a specimen of the subject is derived from the cell type or tissue, wherein the determining is effected by ascertaining the methylation status of at least two methylation sites on a continuous nucleotide sequence of the same double-stranded, cell-free DNA molecule, wherein the continuous nucleotide sequence is no longer than 300 base pairs and is comprised in a sequence as set forth in any one of SEQ ID NOs: 2-117 or 121-177.

According to an aspect of some embodiments of the present invention there is provided a method of determining whether a double-stranded, cell-free DNA molecule is derived from a cell type or tissue of interest in a specimen, the method comprising: ascertaining the methylation status of at least two methylation sites on a continuous nucleotide sequence of the same double-stranded cell-free DNA molecule, wherein the nucleotide sequence comprises no more than 300 base pairs and is comprised in a sequence as set forth in any one of SEQ ID Nos: 2-117 or 121-177, wherein a methylation status of each of the at least two methylation sites on the continuous nucleotide sequence of the double-stranded, cell-free DNA molecule characteristic of the cell type or tissue of interest is indicative that the double-stranded, cell-free DNA molecule is derived from the cell type or tissue of interest.

According to an aspect of some embodiments of the present invention there is provided a kit for identifying the source of DNA in a sample comprising oligonucleotides which are capable of detecting the methylation status of at least two methylation sites in a nucleic acid sequence of the same molecule of DNA, the nucleic acid sequence being no longer than 300 base pairs and comprising at least two methylation sites which are differentially methylated in a first cell of interest with respect to a second cell which is non-identical to the first cell of interest, wherein the nucleic acid sequence is comprised in a sequence as set forth in any one of SEQ ID Nos: 2-117 or 121-177.

According to an aspect of some embodiments of the present invention there is provided a kit for identifying the source of DNA in a sample comprising at least two oligonucleotides which are capable of amplifying a DNA molecule having a nucleic acid sequence no longer than 300 base pairs, wherein the nucleic acid sequence comprises at least two methylation sites which are differentially methylated in a first cell of interest with respect to a second cell which is non-identical to the first cell of interest, wherein the nucleic acid sequence is comprised in a sequence as set forth in any one of SEQ ID Nos: 2-117 or 121-177.

According to an aspect of some embodiments of the present invention there is provided a method of classifying a disease associated with tissue damage, the disease being selected from the group consisting of sepsis, lupus and HIV, the method comprising analyzing cell-free DNA derived from the tissue in a fluid sample of the subject, wherein the amount of the cell-free DNA is indicative of a classification of the sepsis, lupus or HIV.

According to an aspect of some embodiments of the present invention there is provided a method of classifying a disease or disorder associated with tissue damage the method comprising analyzing cell-free DNA derived from the tissue in a fluid sample of the subject, wherein the cell-free DNA comprises a sequence which is comprised in any one of SEQ ID NOs: 2-117 or SEQ ID NOs: 121-177, wherein the amount of the cell-free DNA is indicative of a classification of the disease or disorder.

According to some embodiments of the invention, the DNA molecule is no longer than 300 base pairs (bp).

According to some embodiments of the invention, the DNA molecule is no longer than 150 bp.

According to some embodiments of the invention, the at least two methylation sites are not more than 300 bp apart on a single strand of the DNA molecule.

According to some embodiments of the invention, the at least two methylation sites are not more than 150 bp apart on a single strand of the DNA molecule.

According to some embodiments of the invention, the at least two methylation sites comprises at least three methylation sites.

According to some embodiments of the invention, the at least two methylation sites comprises at least four methylation sites.

According to some embodiments of the invention, the at least three methylation sites are not more than 300 bp apart on a single strand of the DNA molecule.

According to some embodiments of the invention, the at least three methylation sites are not more than 150 bp apart on a single strand of the DNA molecule.

According to some embodiments of the invention, the at least four methylation sites are not more than 300 bp apart on a single strand of the DNA molecule.

According to some embodiments of the invention, the at least four methylation sites are not more than 150 bp apart on a single strand of the DNA molecule.

According to some embodiments of the invention, the methylation status is characteristic of a non-diseased cell type or tissue of interest.

According to some embodiments of the invention, the specimen is a fluid sample selected from the group consisting of blood, plasma, sperm, milk, urine, saliva and cerebral spinal fluid.

According to some embodiments of the invention, the ascertaining is effected using at least one methylation-dependent oligonucleotide.

According to some embodiments of the invention, the ascertaining is effected using at least one methylation-independent oligonucleotide.

According to some embodiments of the invention, the ascertaining is effected using at least one methylation-independent oligonucleotide which targets the forward strand of the double-stranded, cell-free DNA molecule and at least one methylation-independent oligonucleotide which targets the reverse strand of the double-stranded, cell-free DNA molecule.

According to some embodiments of the invention, the ascertaining is effected using digital droplet PCR.

According to some embodiments of the invention, the ascertaining is effected by contacting the DNA molecule in the sample with bisulfite to generate single-stranded DNA molecules of which demethylated cytosines of the single-stranded DNA molecules are converted to uracils.

According to some embodiments of the invention, the method further comprises contacting the single-stranded DNA with amplification primers under conditions that generate amplified DNA from the single-stranded DNA following the contacting with the bisulfite.

According to some embodiments of the invention, the method further comprises sequencing the amplified DNA.

According to some embodiments of the invention, the contacting is effected using at least two non-identical labels.

According to some embodiments of the invention, the determining is effected using a single label.

According to some embodiments of the invention, the ascertaining comprises:
(a) contacting the amplified DNA with:
   (i) a first probe that hybridizes to the amplified DNA at a site which comprises the first of the at least two methylation sites; and
   (ii) a second probe that hybridizes to the amplified DNA at a site which comprises a second of the at least two methylation sites, wherein the first probe and the second probe are labeled with non-identical detectable moieties, wherein the first probe and the second probe comprise a quenching moiety;
   wherein the contacting is effected under conditions that separate the quenching moiety from the first probe and the second probe to generate a non-quenched first probe and a non-quenched second probe; and
(b) analyzing the amount of the non-quenched first probe and the non-quenched second probe in at least one specimen fraction of the plurality of specimen fractions.

According to some embodiments of the invention, the first probe hybridizes to the forward strand of the amplified DNA and the second probe hybridizes to the reverse strand of the amplified DNA.

According to some embodiments of the invention, the specimen comprises cell-free DNA which is derived from a second cell which is non-identical to the cell type or tissue.

According to some embodiments of the invention, the method further comprises analyzing the amount of cell-free DNA derived from the cell type or tissue: amount of cell-free DNA derived from the second cell.

According to some embodiments of the invention, the method further comprises analyzing the amount of cell-free DNA derived from the cell type or tissue: total amount of cell-free DNA in the sample.

According to some embodiments of the invention, the cell type is selected from the group consisting of a pancreatic beta cell, a pancreatic exocrine cell, a hepatocyte, a brain cell, a lung cell, a uterus cell, a kidney cell, a breast cell, an adipocyte, a colon cell, a rectum cell, a cardiac cell, a skeletal muscle cell, a prostate cell and a thyroid cell.

According to some embodiments of the invention, the tissue is selected from the group consisting of pancreatic tissue, liver tissue, lung tissue, brain tissue, uterus tissue, renal tissue, breast tissue, fat, colon tissue, rectum tissue, heart tissue, skeletal muscle tissue, prostate tissue and thyroid tissue.

According to some embodiments of the invention, the specimen is a blood sample.

According to some embodiments of the invention, the method further comprises quantitating the amount of cell-free DNA which is derived from the cell type or tissue.

According to some embodiments of the invention, the method further comprises quantifying the amount of DNA molecules having a methylation status at the continuous sequence characteristic of the cell type or tissue following the ascertaining.

According to some embodiments of the invention, the ascertaining is effected using a multiplex reaction.

According to some embodiments of the invention, the DNA is cell-free DNA.

According to some embodiments of the invention, the kit further comprises at least one agent for sequencing the DNA sequence.

According to some embodiments of the invention, the kit further comprises DNA having the nucleic acid sequence, wherein the DNA is derived from a known cell of interest.

According to some embodiments of the invention, the kit further comprises bisulfite.

According to some embodiments of the invention, the at least one of the oligonucleotides encodes a bar-code sequence and/or is labeled with a detectable moiety.

According to some embodiments of the invention, the kit further comprises
(i) a Taqman polymerase; and/or
(ii) a droplet forming oil.

According to some embodiments of the invention, the cell-free DNA is derived from cardiac tissue or hepatic tissue.

According to some embodiments of the invention, the disease or disorder is selected from the group consisting of sepsis, lupus, myocardial infarction and HIV.

According to some embodiments of the invention, the tissue is cardiac tissue or hepatic tissue.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### The invention can also be understood with reference to the following numbered clauses.

1. A method of ascertaining the methylation status of a double-stranded, cell-free DNA molecule in a specimen, the method comprising ascertaining the methylation status of at least two methylation sites of the same double-stranded cell-free DNA molecule, wherein said double-stranded, cell-free DNA molecule comprises a nucleotide sequence which comprises no more than 300 base pairs and is comprised in a sequence as set forth in any one of SEQ ID NOs: 2-117 or 121-177, thereby ascertaining the methylation status of a double-stranded, cell-free DNA molecule.
2. A method of detecting death of a cell type or tissue in a subject comprising determining whether a double-stranded, cell-free DNA molecule comprised in a specimen of the subject is derived from the cell type or tissue, wherein said determining is effected by ascertaining the methylation status of at least two methylation sites on a continuous nucleotide sequence of the same double-stranded, cell-free DNA molecule, wherein said continuous nucleotide sequence is no longer than 300 base pairs and is comprised in a sequence as set forth in any one of SEQ ID NOs: 2-117 or 121-177.
3. A method of determining whether a double-stranded, cell-free DNA molecule is derived from a cell type or tissue of interest in a specimen, the method comprising: ascertaining the methylation status of at least two methylation sites on a continuous nucleotide sequence of the same double-stranded cell-free DNA molecule, wherein said nucleotide sequence comprises no more than 300 base pairs and is comprised in a sequence as set forth in any one of SEQ ID Nos: 2-117 or 121-177, wherein a methylation status of each of said at least two methylation sites on said continuous nucleotide sequence of said double-stranded, cell-free DNA molecule characteristic of said cell type or tissue of interest is indicative that the double-stranded, cell-free DNA molecule is derived from the cell type or tissue of interest.
4. The method of clauses 2 or 3, wherein the DNA molecule is no longer than 300 base pairs (bp).
5. The method of any one of clauses 1-3, wherein said DNA molecule is no longer than 150 bp.
6. The method of any one of clauses 1-5, wherein said at least two methylation sites are not more than 300 bp apart on a single strand of the DNA molecule.
7. The method of any one of clauses 1-6, wherein said at least two methylation sites are not more than 150 bp apart on a single strand of the DNA molecule.
8. The method of any one of clauses 1-3, wherein said at least two methylation sites comprises at least three methylation sites.
9. The method of any one of clauses 1-3, wherein said at least two methylation sites comprises at least four methylation sites.
10. The method of clause 8, wherein said at least three methylation sites are not more than 300 bp apart on a single strand of the DNA molecule.
11. The method of clause 8, wherein said at least three methylation sites are not more than 150 bp apart on a single strand of the DNA molecule.
12. The method of clause 9, wherein said at least four methylation sites are not more than 300 bp apart on a single strand of the DNA molecule.
13. The method of clause 9, wherein said at least four methylation sites are not more than 150 bp apart on a single strand of the DNA molecule.
14. The method of any one of clauses 1-3, wherein said methylation status is characteristic of a non-diseased cell type or tissue of interest.
15. The method of any one of clauses 1-3, wherein said specimen is a fluid sample selected from the group consisting of blood, plasma, sperm, milk, urine, saliva and cerebral spinal fluid.
16. The method of any one of clauses 1-3, wherein said ascertaining is effected using at least one methylation-dependent oligonucleotide.
17. The method of any one of clauses 1-3, wherein said ascertaining is effected using at least one methylation-independent oligonucleotide.
18. The method of clause 17, wherein said ascertaining is effected using at least one methylation-independent oligonucleotide which targets the forward strand of the double-stranded, cell-free DNA molecule and at least one methylation-independent oligonucleotide which targets the reverse strand of the double-stranded, cell-free DNA molecule.
19. The method of any one of clauses 1-3, wherein said ascertaining is effected using digital droplet PCR.
20. The method of any one of clauses 1-3, wherein said ascertaining is effected by contacting the DNA molecule in the sample with bisulfite to generate single-stranded DNA molecules of which demethylated cytosines of said single-stranded DNA molecules are converted to uracils.
21. The method of clause 20, further comprising contacting said single-stranded DNA with amplification primers under conditions that generate amplified DNA from said single-stranded DNA following said contacting with said bisulfite.
22. The method of clause 21, further comprising sequencing said amplified DNA.
23. The method of clause 21, wherein said contacting is effected using at least two non-identical labels.
24. The method of clause 21, wherein said determining is effected using a single label.
25. The method of clause 21, wherein said ascertaining comprises:
   (a) contacting said amplified DNA with:
      (i) a first probe that hybridizes to said amplified DNA at a site which comprises the first of said at least two methylation sites; and
      (ii) a second probe that hybridizes to said amplified DNA at a site which comprises a second of said at least two methylation sites, wherein said first probe and said second probe are labeled with non-identical detectable moieties, wherein said first probe and said second probe comprise a quenching moiety;
      wherein said contacting is effected under conditions that separate said quenching moiety from said first probe and said second probe to generate a non-quenched first probe and a non-quenched second probe; and
   (b) analyzing the amount of said non-quenched first probe and said non-quenched second probe in at least one specimen fraction of a plurality of specimen fractions.
26. The method of clause 25, wherein said first probe hybridizes to the forward strand of said amplified DNA and said second probe hybridizes to the reverse strand of said amplified DNA.
27. The method of any one of clauses 1-3, wherein the specimen comprises cell-free DNA which is derived from a second cell which is non-identical to said cell type or tissue.
28. The method of clause 27, further comprising analyzing the amount of cell-free DNA derived from said cell type or tissue: amount of cell-free DNA derived from said second cell.
29. The method of clause 27, further comprising analyzing the amount of cell-free DNA derived from said cell type or tissue: total amount of cell-free DNA in the sample.
30. The method of clauses 2 or 3, wherein said cell type is selected from the group consisting of a pancreatic beta cell, a pancreatic exocrine cell, a hepatocyte, a brain cell, a lung cell, a uterus cell, a kidney cell, a breast cell, an adipocyte, a colon cell, a rectum cell, a cardiac cell, a skeletal muscle cell, a prostate cell and a thyroid cell.
31. The method of clauses 2 or 3, wherein said tissue is selected from the group consisting of pancreatic tissue, liver tissue, lung tissue, brain tissue, uterus tissue, renal tissue, breast tissue, fat, colon tissue, rectum tissue, heart tissue, skeletal muscle tissue, prostate tissue and thyroid tissue.
32. The method of any one of clauses 1-3, wherein the specimen is a blood sample.
33. The method of clause 2, further comprising quantitating the amount of cell-free DNA which is derived from said cell type or tissue.
34. The method of clauses 2 or 3, further comprising quantifying the amount of DNA molecules having a methylation status at said continuous sequence characteristic of said cell type or tissue following said ascertaining.
35. The method of any one of clauses 1-3, wherein said ascertaining is effected using a multiplex reaction.
36. A kit for identifying the source of DNA in a sample comprising oligonucleotides which are capable of detecting the methylation status of at least two methylation sites in a nucleic acid sequence of the same molecule of DNA, said nucleic acid sequence being no longer than 300 base pairs and comprising at least two methylation sites which are differentially methylated in a first cell of interest with respect to a second cell which is non-identical to said first cell of interest, wherein said nucleic acid sequence is comprised in a sequence as set forth in any one of SEQ ID Nos: 2-117 or 121-177.
37. A kit for identifying the source of DNA in a sample comprising at least two oligonucleotides which are capable of amplifying a DNA molecule having a nucleic acid sequence no longer than 300 base pairs, wherein said nucleic acid sequence comprises at least two methylation sites which are differentially methylated in a first cell of interest with respect to a second cell which is non-identical to said first cell of interest, wherein said nucleic acid sequence is comprised in a sequence as set forth in any one of SEQ ID Nos: 2-117 or 121-177.
38. The kit of clause 36, wherein said DNA is cell-free DNA.
39. The kit of clause 36 or 37, further comprising at least one agent for sequencing said DNA sequence.
40. The kit of clauses 36 or 37, further comprising DNA having said nucleic acid sequence, wherein said DNA is derived from a known cell of interest.
41. The kit of clauses 36 or 37, further comprising bisulfite.
42. The kit of clauses 36 or 37, wherein at least one of said oligonucleotides encodes a bar-code sequence and/or is labeled with a detectable moiety.
43. The kit of any one of clauses 36 or 37, further comprising:
   (i) a Taqman polymerase; and/or
   (ii) a droplet forming oil.
44. A method of classifying a disease associated with tissue damage, said disease being selected from the group consisting of sepsis, lupus and HIV, the method comprising analyzing cell-free DNA derived from said tissue in a fluid sample of the subject, wherein the amount of said cell-free DNA is indicative of a classification of the sepsis, lupus or HIV.
45. The method of clause 44, wherein said cell-free DNA is derived from cardiac tissue or hepatic tissue.
46. A method of classifying a disease or disorder associated with tissue damage the method comprising analyzing cell-free DNA derived from said tissue in a fluid sample of the subject, wherein said cell-free DNA comprises a sequence which is comprised in any one of SEQ ID NOs: 2-117 or SEQ ID NOs: 121-177, wherein the amount of said cell-free DNA is indicative of a classification of the disease or disorder.
47. The method of clause 46, wherein said disease or disorder is selected from the group consisting of sepsis, lupus, myocardial infarction and HIV.
48. The method of clause 46, wherein said tissue is cardiac tissue or hepatic tissue.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

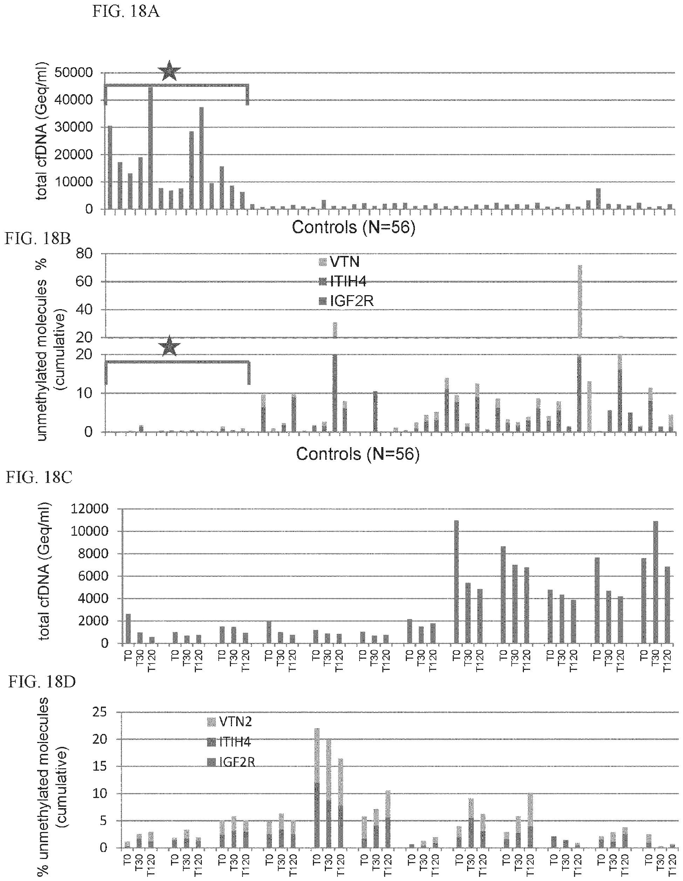

In the drawings:

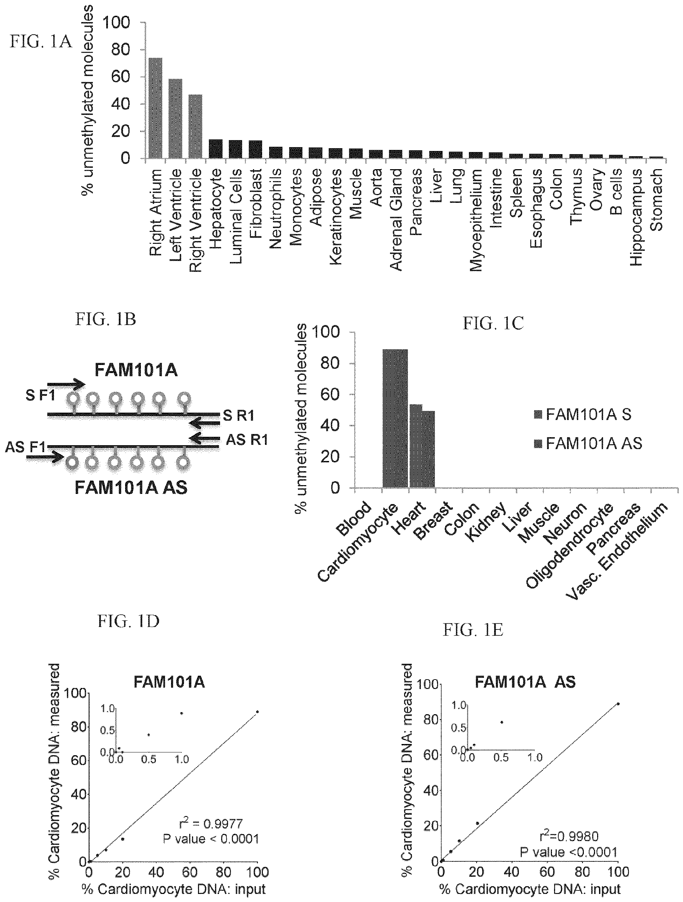

FIGs. 1A-E: Identification of cardiomyocyte-specific DNA methylation markers.
   1A. Unmethylation levels of FAM101A locus in 27 human tissues, including left ventricle, right ventricle and right atrium (red). Data was extracted from the Roadmap Epigenomics Consortium browser.
   1B. Structure of the FAM101A locus, used as two independent markers: FAM101A and FAM101A AS. Lollipops represent CpG sites; arrows mark positions of PCR primers; S, sense marker; AS, antisense marker.
   1C. Unmethylation status of FAM101A and FAM101A AS in DNA from multiple tissues and from isolated cardiomyocytes (purchased from ScienCell Research Laboratories, San Diego, CA). Targeted PCR yields a lower background in non cardiac tissues compared with the Roadmap browser in panel A, since the roadmap data includes molecules that contain only some of the cytosines in the FAM101A locus (e.g. only one or two), which can occasionally be demethylated in non-cardiac tissue. In contrast, the targeted PCR by definition amplifies only molecules containing all cytosines in the locus.
   1D-E. Spike in experiments for FAM101A and FAM101A AS. Human cardiomyocyte DNA was mixed with human leukocyte DNA in the indicated proportions (0- 100%), and the percentage of fully unmethylated *FAM101A* molecules (in which all five CpG sites were converted by bisulfite) was determined.

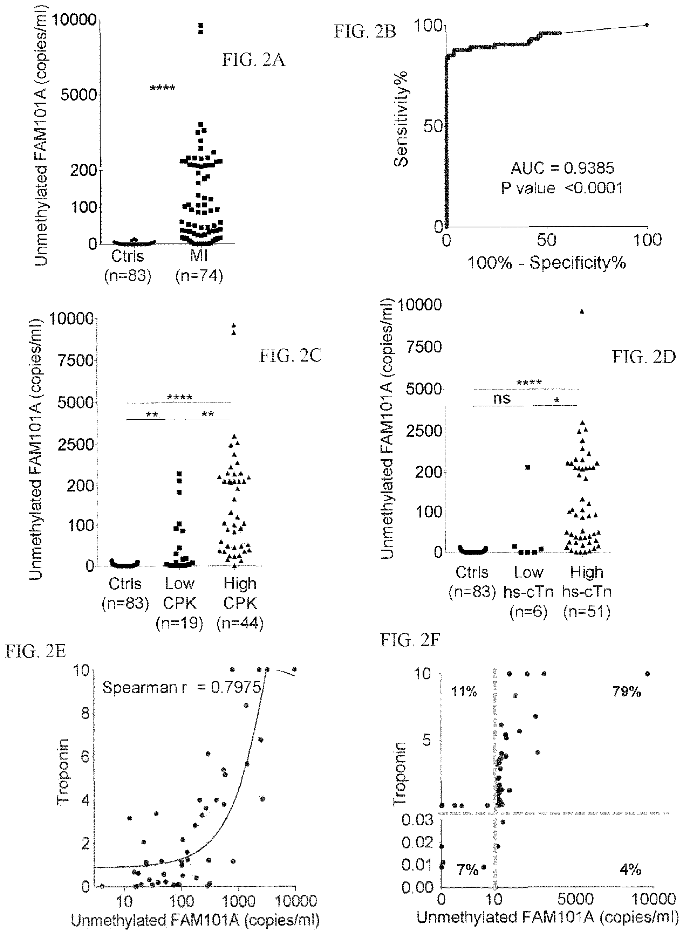

FIGs. 2A-F: Cardiomyocyte-derived cfDNA in healthy subjects and in patients with myocardial infarction.
   A. Cardiac cfDNA (copies of fully unmethylated FAM101A /ml plasma) in samples from healthy controls (n=61) and patients during MI (n=79). Mann-Whitney test for controls vs. patients, *P* <0.0001
   B. Receiver operating characteristic (ROC) curve for unmethylated FAM101A levels in healthy controls and patients with MI. Area under the curve (AUC) 0.884 (95% CI=0.8925 to 0.9766)
   C. Comparison of unmethylated FAM101A levels (copies/ml) in samples from healthy controls, MI patients with low Creatine Kinase (CPK <200) and MI patients with high CK (CK>200). Kruskal-Wallis test P value<0.0001. Dunn's multiple comparisons test adjusted P Value: Ctrls vs. low CK, p< 0.001; Ctrls vs. high CK, P<0.0001; low CK vs. high CK, P=0.0064.
   D. Comparison of unmethylated FAM101A levels in samples from healthy controls, MI patients with low levels of high-sensitive troponin T (hs-cTn) (<0.03), and MI patients with high levels of hs-cTn (>0.03). Dunn's multiple comparisons test adjusted P Value: Ctrls vs. low hs-cTn (<0.03), P=0.8645; Ctrls vs. high hs-cTn (>0.03), PV<0.0001; low hs-cTn (<0.03) vs. high hs-cTn (>0.03), P=0.0189.
   E. Spearman correlation between cardiac cfDNA and troponin levels in n=57 samples.
   F. XY Scatter plot for cardiac cfDNA levels vs. cardiac troponin. Quadrants indicate negative and positive hs-Tn, and negative and positive cardiac cfDNA. Numbers indicate the percentage of samples in each quadrant.
FIGs. 3A-C: Cardiac cfDNA dynamics during MI and after angioplasty.
   A. Cardiac cfDNA levels in MI patients before and after PCI.
   B. ROC curve for cardiac cfDNA in healthy individuals versus MI patients prior to intervention.
   C. Time course of cardiac cfDNA and troponin levels in five patients. Vertical dashed lines indicate PCI time.
FIGs. 4A-C: Cardiac cfDNA in sepsis.
   A. Levels of cardiac cfDNA in healthy controls and patients with sepsis.
   B. Lack of correlation between cardiac cfDNA and troponin. Curved line represents non linear (quadratic) fit. C.Kaplan-Meier plot showing correlation of cardiac cfDNA to patient survival.
FIGs. 5A-D: detection of cardiac cfDNA using digital droplet PCR.
   A. Schematic of approach for ddPCR-based detection of methylation status of multiple adjacent cytosines. A signal from two probes in the same droplet reflects lack of methylation in 5 adjacent cytosines in the same original DNA strand.
   B. Signal from cardiomyocyte and leukocyte DNA based on individual or dual probes. Scoring only dual probe signals drastically reduces noise from leukocyte DNA.
   C. Spike-in experiment assessing sensitivity and linearity of signal from cardiomyocyte DNA diluted in leukocyte DNA. The use of dual probe enhances linearity and reduces baseline signal.
   D. Measurement of cardiac cfDNA in plasma of healthy adult and patients with myocardial infarction. The use of dual probes reduces the baseline signal in healthy plasma.

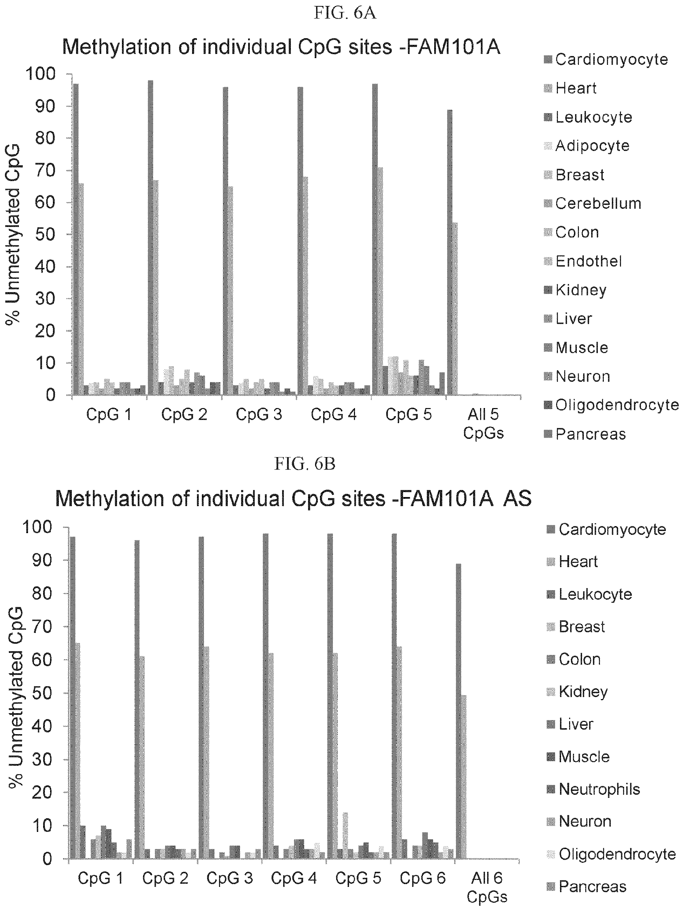

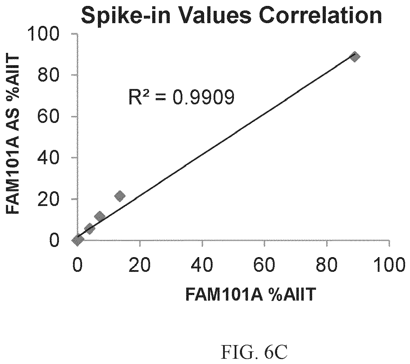

FIGs. 6A-C: methylation of individual and multiple adjacent cytosines within the FAM101A locus.
   A. Methylation status of cytosines in the sense strand of FAM101A
   B. Metylation status of cytosines in the antisense (AS) strand of FAM101A. Graphs shows the percentage of unmethylated molecules in DNA from each tissue. The set of columns on the far right describes the percentage of molecules in which all CpG sites are unmethylated, demonstrating the higher in signal-to-noise ratio afforded by interrogating all CpGs simultaneously.
   C. Correlation between results of spike-in experiments using the sense and antisense FAM101A markers.

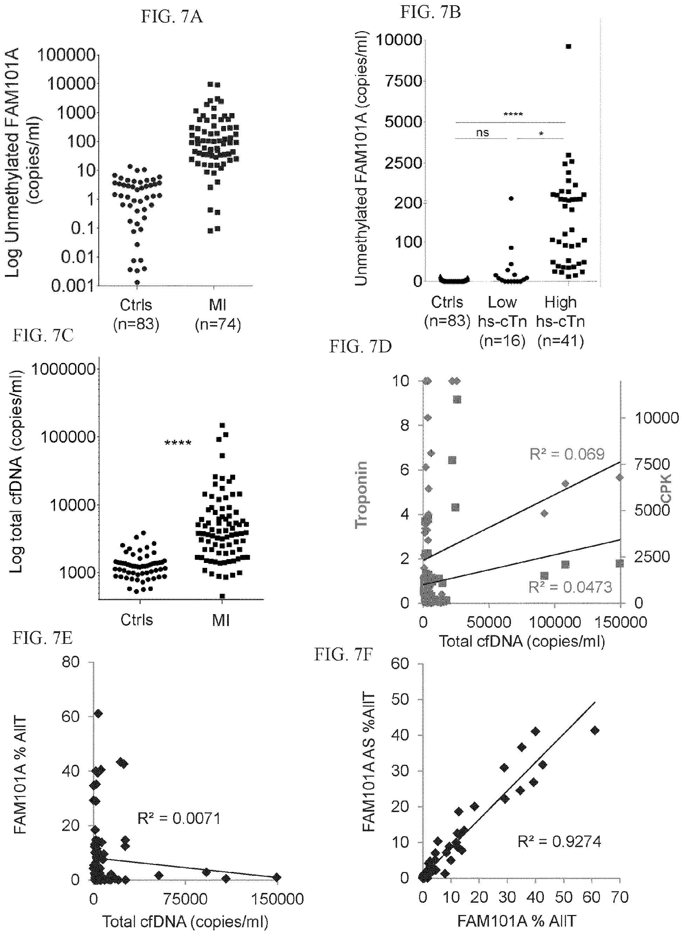

FIGs. 7A-F: additional correlations of cardiac and total cfDNA in MI patients.
   A. Log scale presentation of unmethylated FAM101A levels in plasma samples from healthy controls (n=83) and patients during MI (n=74). 54 values were zero, so are not shown in the graph.
   B. Cardiac cfDNA levels in controls vs MI patients positive or negative for high sensitive troponin using 0.1 as a cutoff. Dunn's multiple comparisons test adjusted P value: Ctrls vs. Low hs-cTn (<0.1), P=0.0433; Ctrls vs. High hs-cTn (>0.1), P<0.0001; Low hs-cTn (<0.1) vs. High hs-cTn (>0.1), P=0.0003.
   C. Total cfDNA concentration in controls and MI patients.
   D. Lack of correlation between total concentration of cfDNA (genome equivalents/ml) and either hs-Tn (blue) or CK (red) levels.
   E. Lack of correlation between total cfDNA (genome equivalents/ml) and percentage of cardiac cfDNA.
   F. Linear correlation between FAM101A sense (S) and antisense (AS) signal in the MI samples.

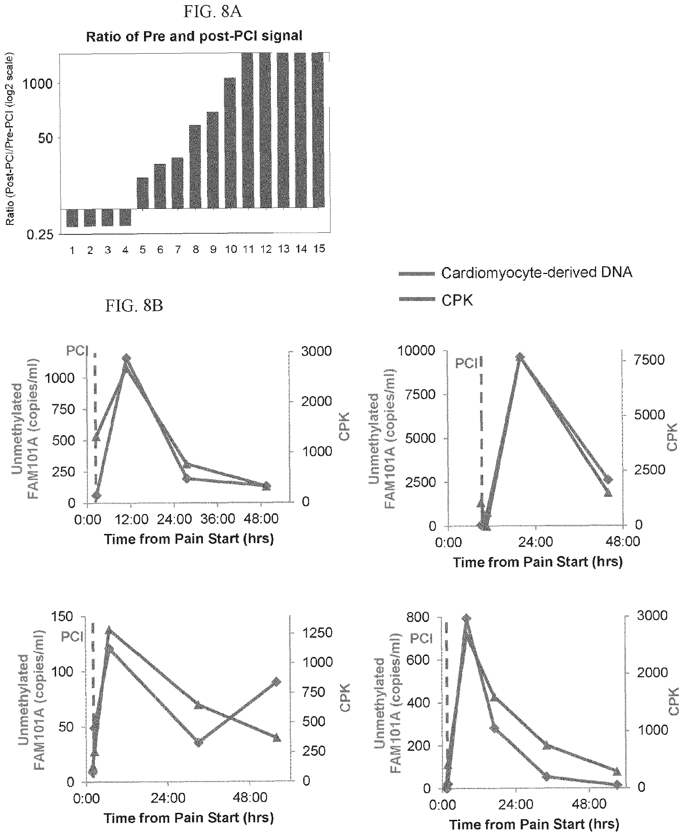

FIGs. 8A-B. Dynamics of cardiac cfDNA and CPK in myocardial infarction.
   A. Ratio of cardiac cfDNA before and after PCI in 15 individuals with MI. As expected, cardiac cfDNA levels increased after intervention.
   B. Dynamics of cardiac cfDNA and CPK in individual patients. Time 0 is the beginning of chest pain. Vertical dashed line indicates time of PCI.

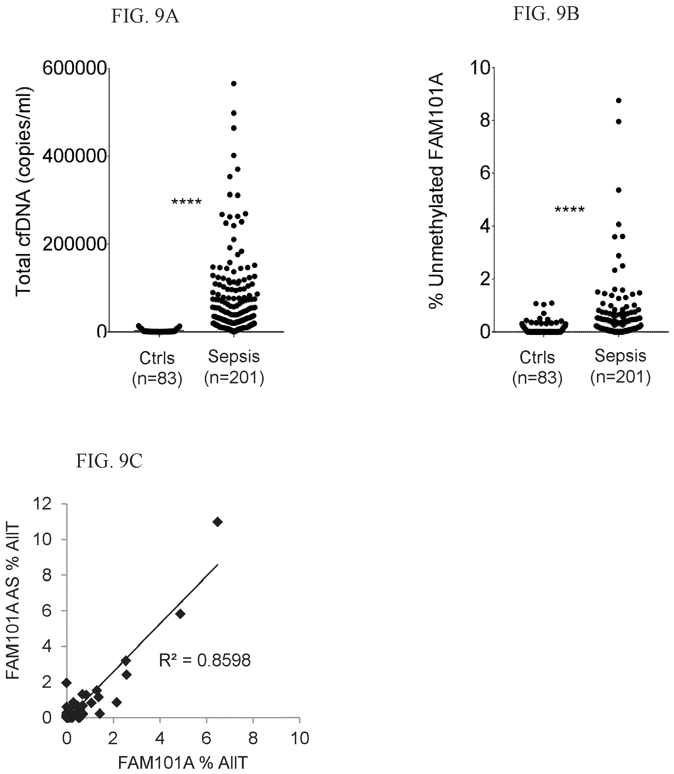

FIGs. 9A-C: Total and cardiac cfDNA levels in patients with sepsis.
   A.Concentration of cfDNA in patients with sepsis.
   B.Percentage of cardiac cfDNA in patients with sepsis.
   C.Correlation between FAM101A sense and antisense signals in sepsis samples.

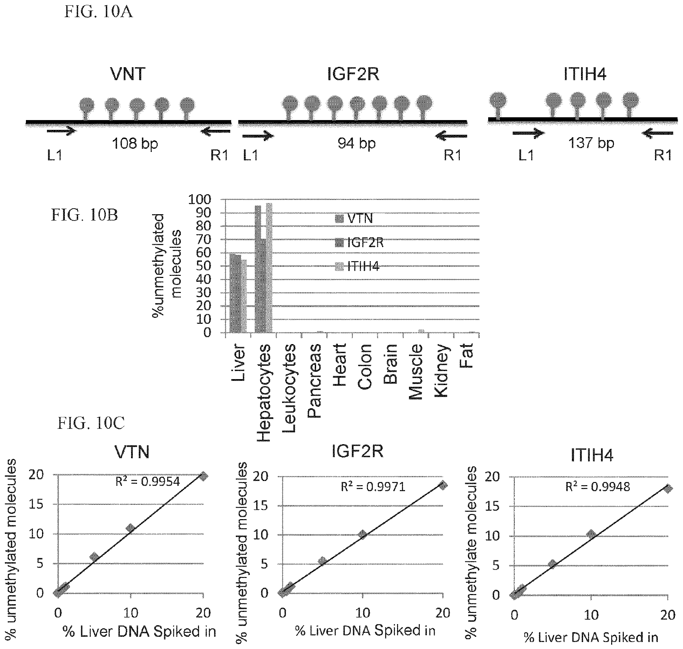

FIGs. 10A-C: Liver specific markers
   A. Structure of the three loci (adjacent to the ITIH4, IGF2R and VTN genes) used as hepatocyte biomarkers. Lollipops represent CpG sites. Red indicates CpG sites represented in the Infinium HumanMethylation450 BeadChip. Arrows mark positions of PCR primers. Markers are defined by the methylation status of CpG sites between primers.
   B. Methylation status of ITIH4, IGF2R, VTN in DNA from multiple tissues. Shown is the percentage of molecules in which all CpG sites were unmethylated.
   C. Spike-in experiments. Human liver DNA was mixed with human leukocyte DNA in the indicated proportions (0 to 20%), and the percentage of fully unmethylated hepatocyte markers was determined.

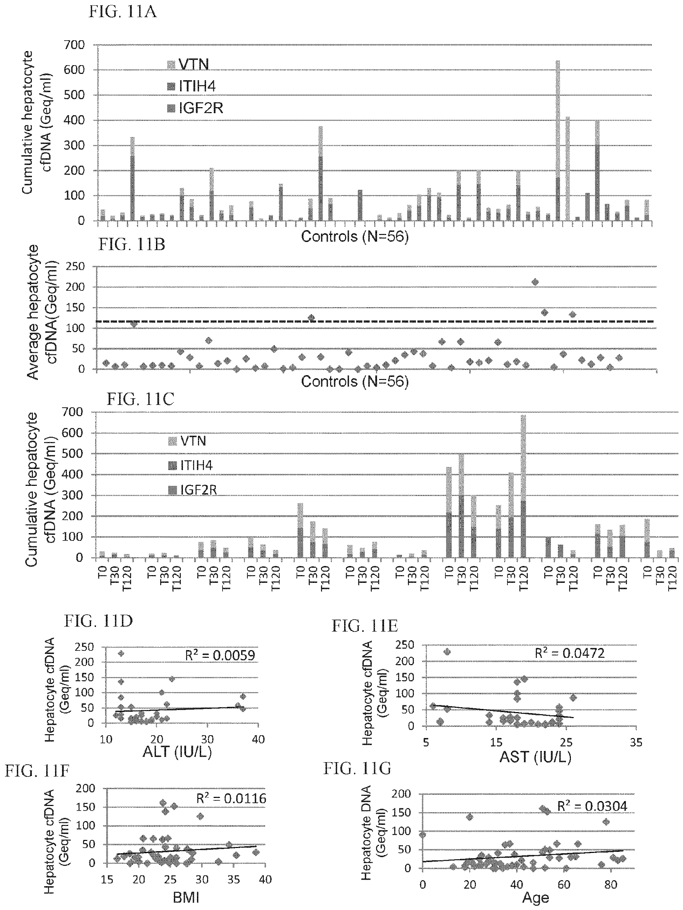

FIGs. 11A-G: Liver-derived cfDNA in healthy individuals
   A. Concentration in genome equivalents (Geq)/ml of hepatocyte derived DNA in the plasma of healthy donors. Green, red and blue indicate the estimation for VTN, ITIH4 and IGF2R markers respectively. The concentration was measured by multiplying the fraction of hepatocyte cfDNA by the concentration of total cfDNA (Figure 18).
   B. Estimation of the concentration of hepatocyte-derived cfDNA in the plasma of healthy donors, averaging the values for all markers. Each dot represents one individual donor. Dashed line indicates average+ two standard deviations.
   C. Estimation of the concentration of hepatocyte-derived cfDNA in the plasma of healthy donors (n=12) at three time points. T0 - after a twelve hour fast, T30 - half an hour after a meal, T120 - two hours after a meal.
   D-G. Lack of correlation between hepatocyte cfDNA in healthy donors and ALT levels (top left), AST levels (top right), BMI (bottom left) and age (bottom right) of the same donors.

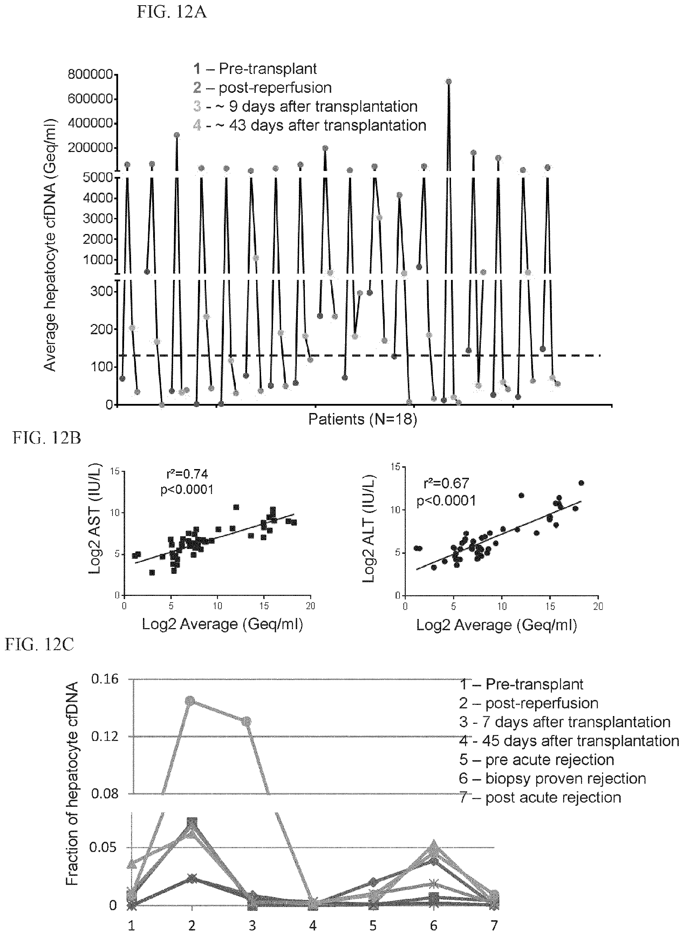

FIGs. 12A-C: Hepatocyte cfDNA in liver transplant recipients.
   A. Hepatocyte-derived DNA in the plasma of 18 liver transplant recipients. Each patient was sampled four time points as indicated. Graph shows the average values of the three liver markers in each sample. Dashed line indicates average+ two standard deviations of healthy controls.
   B. Correlation between hepatocyte cfDNA levels and circulating liver enzymes in liver transplant recipients.
   C. Hepatocyte-derived DNA in the plasma of six liver transplantation patients who had an episode of graft rejection. Each patient was examined at the six time points indicated. Graph shows the average values of the fraction of the three liver markers in each sample. Each line represents the values for one patient.

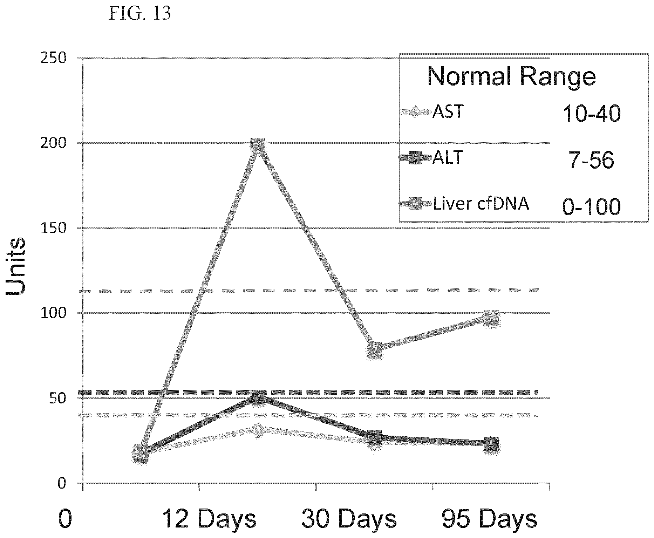

FIG. 13: Identification of hepatocyte-derived cfDNA after partial hepatectomy. Hepatocyte-derived DNA in the plasma of 14 liver donors after partial hepatectomy. Each patient was sampled at four time points as indicated. Graph shows the average values of the three liver markers in each sample (genome equivalents/ml) as well as AST and ALT values. Dashed line indicates average+ two standard deviations of healthy controls.

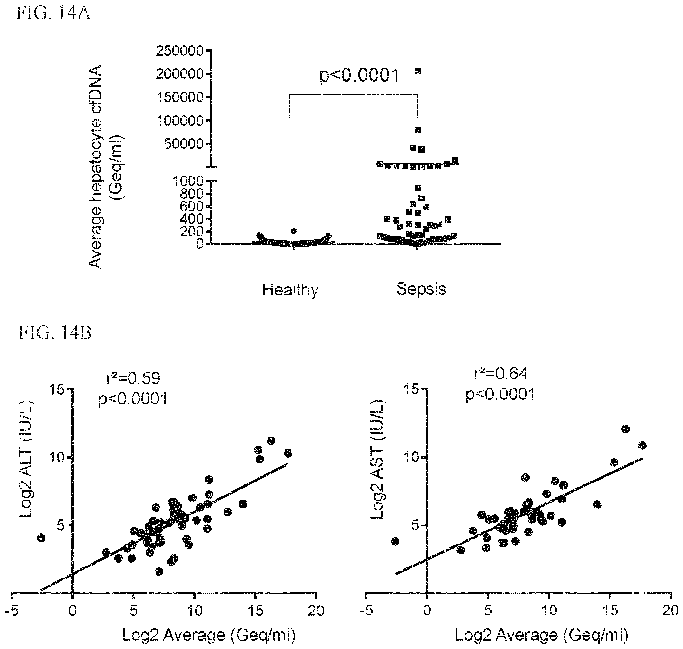

FIGs. 14A-B. Identification of liver derived cfDNA in sepsis.
   A. Hepatocyte cfDNA in the plasma of patients with sepsis
   B. Correlation between hepatocyte cfDNA levels and circulating AST and ALT in septic patients.

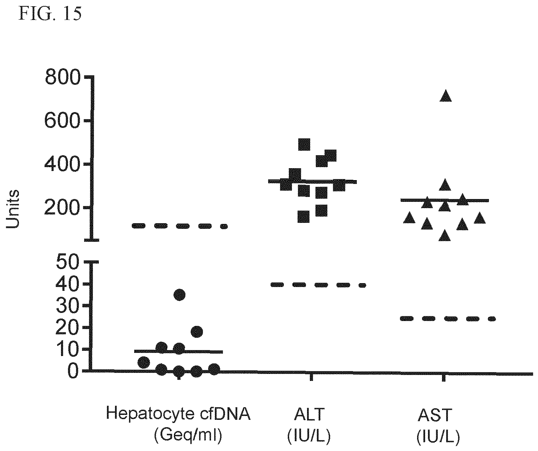

FIG. 15: Identification of hepatocyte-derived cfDNA in DMD. Hepatocyte-derived cfDNA and transaminases in the plasma of 10 DMD patients. Dashed line indicates cutoff for healthy individuals.

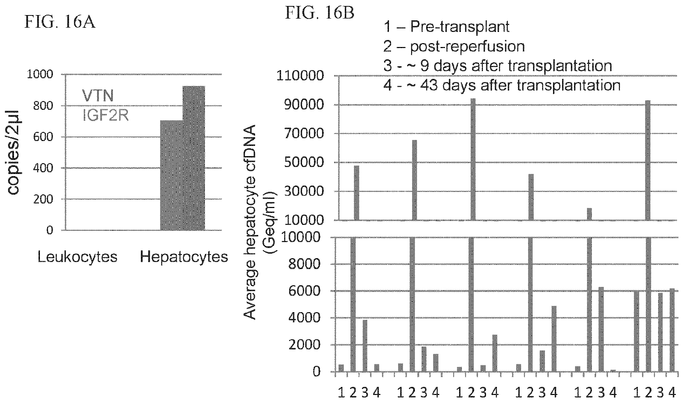

FIGs. 16A-B. Digital droplet PCR for the identification of liver derived cfDNA.
   A. Hepatocyte and leukocyte DNA examined using ddPCR.
   B. Hepatocyte-derived DNA in the plasma of six liver transplant recipients. Each patient was sampled at four time points as indicated. Graph shows the average values of the two liver markers in each sample.

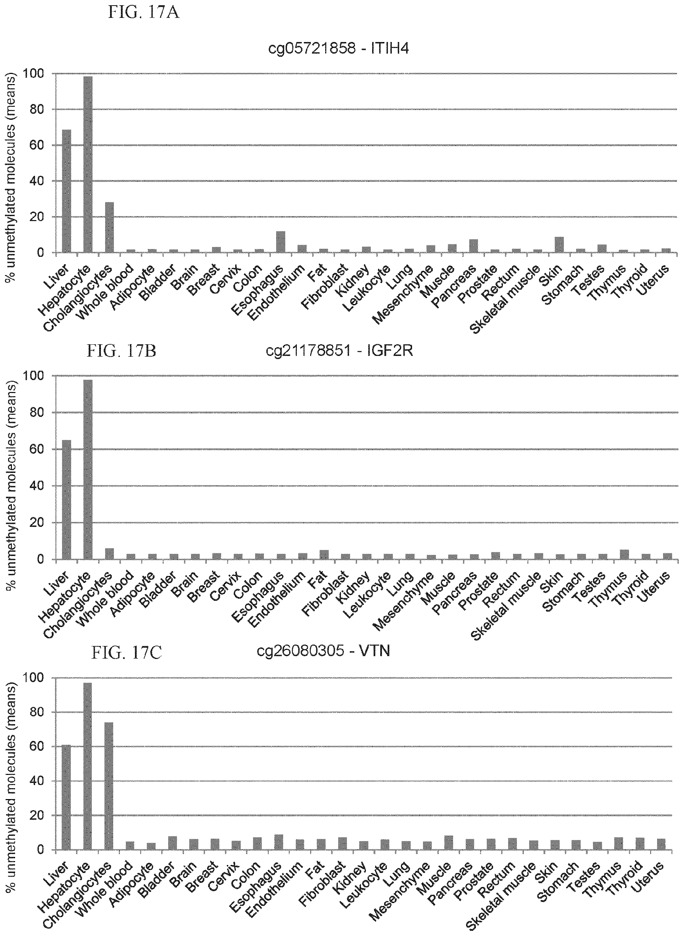

FIGs. 17A-C. Identification of liver-specific DNA methylation markers using methylome datasets. Methylation status of the individual CpG site at the ITIH4 locus (A), IGF2R locus (B) and VTN locus (C) that is captured in the Illumina 450k array.
FIGs. 18A-D. Liver derived cfDNA in healthy controls
   A. Total cfDNA concentration measured per ml plasma. Star indicates plasma samples that were isolated after a prolonged incubation of blood at room temperature, leading to the release of DNA from lysed leukocytes and therefore increased concentration of total cfDNA.
   B. Percentage of hepatocyte-derived cfDNA in the plasma of healthy controls. Green, red and blue indicate percentage measured by the VTN marker, ITIH4 marker and IGF2R marker respectively. Note that individuals with higher total cfDNA had a lower proportion of hepatocyte cfDNA.
   C. Total cfDNA concentration per ml plasma in healthy individuals sampled at three time points. T0 - after a twelve hour fast, T30 - 30 minutes after a meal, T120 - 2 hours after a meal.
   D. Percentage of hepatocyte-derived cfDNA in the plasma of healthy controls at three time points. Green, red and blue indicate percentage measured by the VTN marker, ITIH4 marker and IGF2R marker respectively.

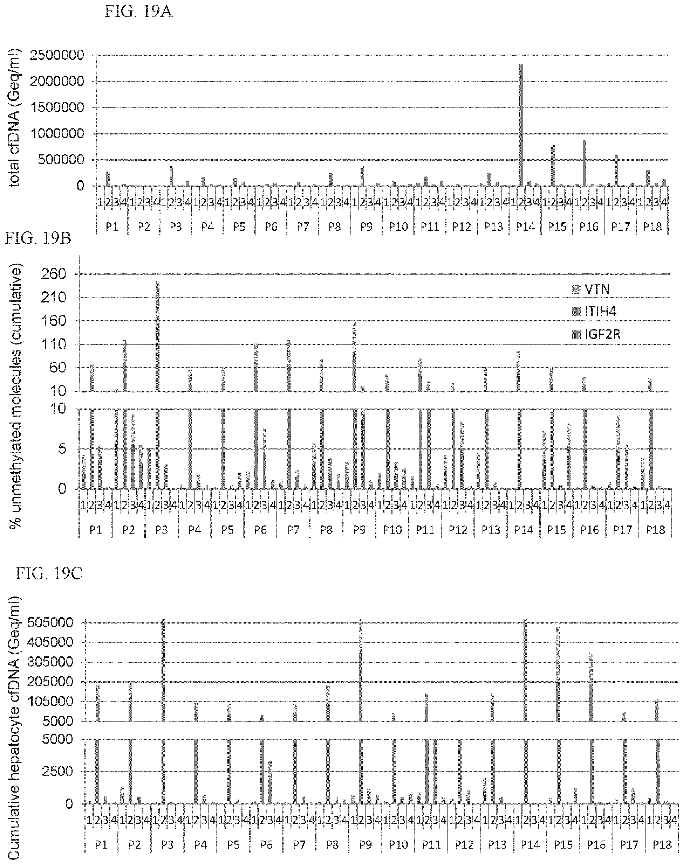

FIGs. 19A-C. Percentage and concentration of hepatocyte cfDNA in liver transplantation patients.
   A. Total cfDNA concentration measured in ml plasma of liver transplant recipients at the indicated time points. 1- pre transplant, 2- post reperfusion, 3- ~9 days after transplantation, 4-~43 days after transplantation.
   B. Percentage of hepatocyte-derived cfDNA in the plasma of liver transplant recipients. Green, red and blue indicate percentages measured by the VTN, ITIH4 and IGF2R markers respectively.
   C. Concentration of hepatocyte-derived cfDNA, calculated by multiplying the fractional value of each marker by the total concentration of cfDNA.

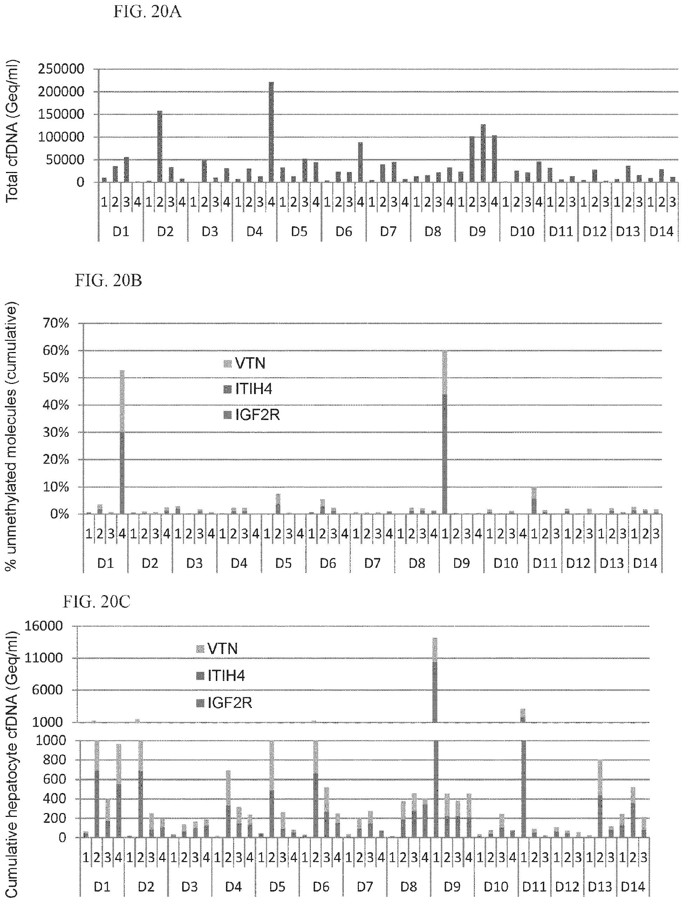

FIGs. 20A-C. Percentage and concentration of liver cfDNA after partial hepatectomy.
   A. Total cfDNA concentration measured per ml plasma. 1- before hepatectomy, 2- 12 days post hepatectomy, 3- 30 days post hepatectomy, 4- 95 days post hepatectomy.
   B. Percentage of hepatocyte-derived cfDNA in the plasma of live donors. Green, red and blue indicating percentages measured by the VTN, ITIH4 and IGF2R markers respectively.
   C. Concentration of hepatocyte-derived cfDNA, calculated by multiplying the fractional value of each marker by the total concentration of cfDNA.

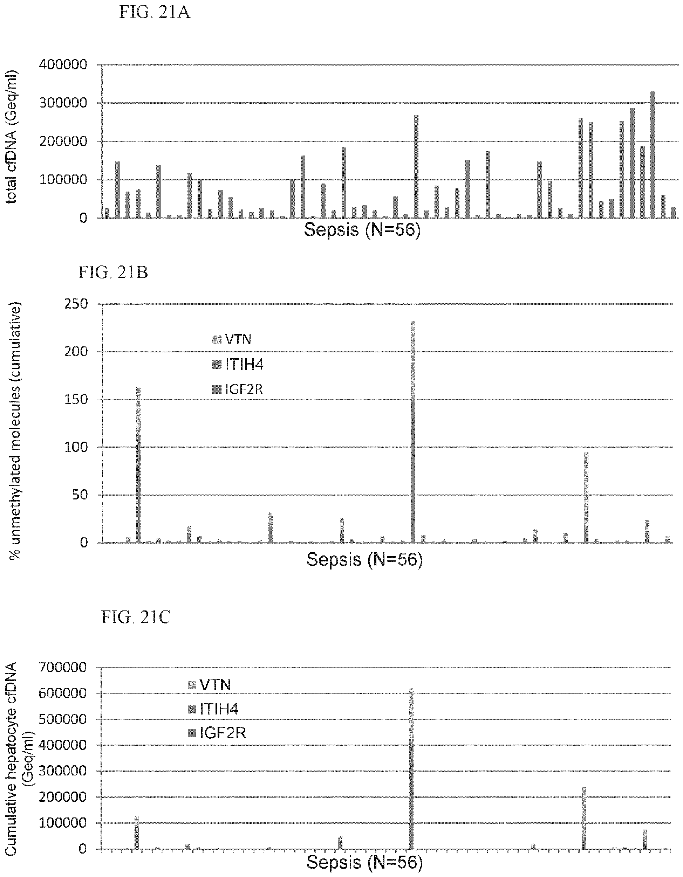

FIGs. 21A-C: Percentage and concentration of liver markers in sepsis patients.
   A. Total cfDNA concentration per ml plasma in sepsis patients
   B.Percentage of hepatocyte-derived cfDNA in sepsis patients. Green, red and blue indicate percentages measured by the VTN, ITIH4 and IGF2R markers respectively.
   C.Concentration of hepatyocyte-derived cfDNA, calculated by multiplying the fractional value of each marker by the total concentration of cfDNA.

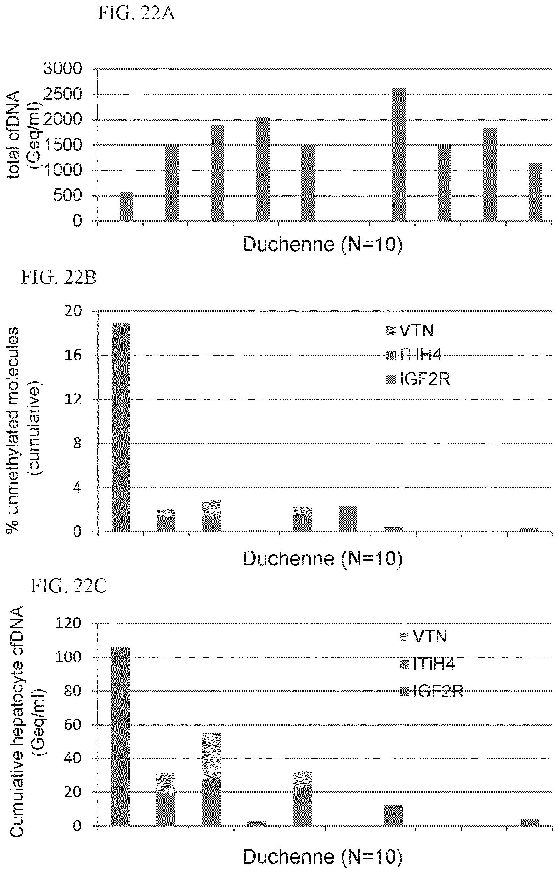

FIGs. 22A-C. Percentage and concentration of liver markers in DMD patients
   A. Total cfDNA concentration per ml plasma in DMD patients
   B. Percentage of hepatocyte-derived cfDNA in DMD patients. Green, red and blue indicate percentages measured by the VTN, ITIH4 and IGF2R marker respectively.
   C. Concentration of hepatocyte-derived cfDNA, calculated by multiplying the fractional value of each marker by the total concentration of cfDNA.

### DESCRIPTION OF SPECIFIC EMBODIMENTS OF THE INVENTION

The present invention contemplates novel target sequences that can be used as tissue-specific methylation markers.

Analysis of circulating DNA is beginning to revolutionize prenatal diagnosis, tumor diagnosis and the monitoring of graft rejection. However a major limitation of all applications is the dependence on the presence of identifiable genetic differences between the tissue of interest and the host.

The present inventors have now identified novel target sequences that can be used to identify cells of interest. Methylation signatures comprising at least two methylation sites comprised in these sequences have been shown to be particularly effective at identifying cell type.

Thus, according to a first aspect of the present invention there is provided a method of ascertaining the methylation status of a double-stranded, cell-free DNA molecule in a specimen, the method comprising ascertaining the methylation status of at least two methylation sites of the same double-stranded cell-free DNA molecule, wherein the double-stranded, cell-free DNA molecule comprises a nucleotide sequence which comprises no more than 300 base pairs and is comprised in a sequence as set forth in any one of SEQ ID NOs: 2-117 or 121-177, thereby ascertaining the methylation status of a double-stranded, cell-free DNA molecule.

As used herein, the term "methylation status" refers to the status of a cytosine in a DNA sequence. The cytosine may be methylated (and present as 5-methylcytosine) or non-methylated and present as cytosine.

As used herein, the term "methylation site" refers to a cytosine residue adjacent to guanine residue (CpG site) that has a potential of being methylated.

The DNA molecule is preferably no longer than 300 nucleotides, 295 nucleotides, 290 nucleotides, 285 nucleotides, 280 nucleotides, 275 nucleotides, 270 nucleotides, 265 nucleotides, 260 nucleotides, 255 nucleotides, 250 nucleotides, 245 nucleotides, 240 nucleotides, 235 nucleotides, 230 nucleotides, 225 nucleotides, 220 nucleotides, 215 nucleotides, 210 nucleotides, 205 nucleotides, 200 nucleotides, 195 nucleotides, 190 nucleotides, 185 nucleotides, 180 nucleotides, 175 nucleotides, 170 nucleotides, 165 nucleotides, 160 nucleotides, 155 nucleotides, 150 nucleotides, 145 nucleotides, 140 nucleotides, 135 nucleotides, 130 nucleotides, 125 nucleotides, 120 nucleotides, 115 nucleotides, 110 nucleotides, 105 nucleotides, 100 nucleotides, 95 nucleotides, 90 nucleotides, 85 nucleotides, 80 nucleotides, 75 nucleotides, 70 nucleotides, 65 nucleotides, 60 nucleotides, 55 nucleotides, or 50 nucleotides.

According to a particular embodiment, the DNA molecule is between 50-300 nucleotides, e.g. between 50-250, between 50-200, between 100-300 nucleotides, or between 100-250 nucleotides.

In another embodiment, the methylation sites of the signature which is analyzed on a double stranded molecule are no more than 300 nucleotides apart, 295 nucleotides apart, 290 nucleotides apart, 285 nucleotides apart, 280 nucleotides apart, 275 nucleotides apart, 270 nucleotides apart, 265 nucleotides apart, 260 nucleotides apart, 255 nucleotides apart, 250 nucleotides apart, 245 nucleotides apart, 240 nucleotides apart, 235 nucleotides apart, 230 nucleotides apart, 225 nucleotides apart, 220 nucleotides apart, 215 nucleotides apart, 210 nucleotides apart, 205 nucleotides apart, 200 nucleotides apart, 195 nucleotides apart, 190 nucleotides apart, 185 nucleotides apart, 180 nucleotides apart, 175 nucleotides apart, 170 nucleotides apart, 165 nucleotides apart, 160 nucleotides apart, 155 nucleotides apart, 150 nucleotides apart, 145 nucleotides apart, 140 nucleotides apart, 135 nucleotides apart, 130 nucleotides apart, 125 nucleotides apart, 120 nucleotides apart, 115 nucleotides apart, 110 nucleotides apart, 105 nucleotides apart, 100 nucleotides apart, 95 nucleotides apart, 90 nucleotides apart, 85 nucleotides apart, 80 nucleotides apart, 75 nucleotides apart, 70 nucleotides apart, 65 nucleotides apart, 60 nucleotides apart, 55 nucleotides apart, or 50 nucleotides apart.

The sequences described herein (SEQ ID NOs: 2-117 and 121-177) comprise sequences which include at least 2, at least 3 or at least 4 methylation sites in a continuous sequence of no more than 300 nucleotides per double stranded DNA molecule. These sequences comprise methylation patterns that are unmethylated in the specified cells and methylated in other cells (e.g. blood cells). According to a particular embodiment, at least one of the methylation sites of the signature are the nucleotides CG which are at position 250 and 251 of each of these sequences.

In accordance with another particular embodiment, the methylation pattern characterizes the normal cell of interest and is not a methylation pattern characterizing a diseased cell (is not for example a methylation pattern characterizing cancer cells of a specific type).

The DNA molecule which is analyzed may comprise at least 2, at least 3 or even at least 4 methylation sites, although at least 5, at least 6, at least 7 at least 8, at least 9 or even at least 10 or more methylation sites are contemplated.

In order to be considered a methylation signature for a particular cell of interest each of the methylation sites of the methylation signature on the DNA molecule should be differentially methylated in that cell of interest with respect to a second non-identical cell. The methylation signature comprises the methylation status of at least two, at least three, at least four methylation sites of a particular DNA molecule. The methylation sites may be on a single strand of the DNA molecule or distributed amongst both strands of the DNA molecule.

According to a particular embodiment, each of the at least two, three or four methylation sites are unmethylated in the cell of interest (the cell for which the methylation pattern is being determined) on the target DNA molecule, whereas in the second non-identical cell each of the sites are methylated on the target DNA molecule.

According to another embodiment, at least one of the methylation sites of the methylation signature is unmethylated in the cell of interest on the DNA molecule, whereas in the second non-identical cell that site is methylated on the DNA molecule.

According to another embodiment, at least two methylation sites of the methylation signature are unmethylated in the cell of interest on the DNA molecule, whereas in the second non-identical cell those sites are methylated on the DNA molecule.

According to another embodiment, at least three methylation sites of the methylation signature are unmethylated in the cell of interest on the DNA molecule, whereas in the second non-identical cell those sites are methylated on the DNA molecule.

According to another embodiment, at least four methylation sites of the methylation signature are unmethylated in the cell of interest on the DNA molecule, whereas in the second non-identical cell those sites are methylated on the DNA molecule.

The second non-identical cell may be of any source including for example blood cells.

The method can be used for identifying methylation signatures of any cell of interest, including but not limited to cardiac cells (e.g. cardiomyocytes), pancreatic cells (such as pancreatic beta cells, exocrine pancreatic cells (e.g. acinar cells), brain cells, oligodendrocytes, liver cells (hepatocytes), kidney cells, tongue cells, vascular endothelial cells, lymphocytes, neutrophils, melanocytes, T-regs, lung cells, a uterus cells, breast cells, adipocytes, colon cells, rectum cells, prostate cells, thyroid cells and skeletal muscle cells. Samples which may be analyzed are generally fluid samples derived from mammalian subjects and include for example blood, plasma, sperm, milk, urine, saliva or cerebral spinal fluid.

Samples which are analyzed typically comprise DNA from at least one, or at least two cell/tissue sources, as further described herein below. Thus for example the samples may comprise cell-free DNA from a single cell type or at least two cell types.

According to a particular embodiment, the sample is plasma or blood.

According to one embodiment, a sample of blood is obtained from a subject according to methods well known in the art. Plasma or serum may be isolated according to methods known in the art.

DNA may be isolated from the blood immediately or within 1 hour, 2 hours, 3 hours, 4 hours, 5 hours or 6 hours. Optionally the blood is stored at temperatures such as 4 °C, or at -20 °C prior to isolation of the DNA. In some embodiments, a portion of the blood sample is used in accordance with the invention at a first instance of time whereas one or more remaining portions of the blood sample (or fractions thereof) are stored for a period of time for later use.

According to one embodiment, the DNA molecule which is analyzed is cellular DNA (i.e. comprised in a cell).

According to still another embodiment, the DNA molecule which is analyzed is comprised in a shedded cell or non-intact cell.

Methods of DNA extraction are well-known in the art. A classical DNA isolation protocol is based on extraction using organic solvents such as a mixture of phenol and chloroform, followed by precipitation with ethanol (J. Sambrook et al., "Molecular Cloning: A Laboratory Manual", 1989, 2nd Ed., Cold Spring Harbour Laboratory Press: New York, N.Y.). Other methods include: salting out DNA extraction (P. Sunnucks et al., Genetics, 1996, 144: 747-756; S. M. Aljanabi and I. Martinez, Nucl. Acids Res. 1997, 25: 4692-4693), trimethylammonium bromide salts DNA extraction (S. Gustincich et al., BioTechniques, 1991, 11: 298-302) and guanidinium thiocyanate DNA extraction (J. B. W. Hammond et al., Biochemistry, 1996, 240: 298-300).

There are also numerous versatile kits that can be used to extract DNA from tissues and bodily fluids and that are commercially available from, for example, BD Biosciences Clontech (Palo Alto, Calif.), Epicentre Technologies (Madison, Wis.), Gentra Systems, Inc. (Minneapolis, Minn.), MicroProbe Corp. (Bothell, Wash.), Organon Teknika (Durham, N.C.), and Qiagen Inc. (Valencia, Calif.). User Guides that describe in great detail the protocol to be followed are usually included in all these kits. Sensitivity, processing time and cost may be different from one kit to another. One of ordinary skill in the art can easily select the kit(s) most appropriate for a particular situation.

According to another embodiment, the DNA which is analyzed is cell-free DNA. For this method, cell lysis is not performed on the sample. Methods of isolating cell-free DNA from body fluids are also known in the art. For example Qiaquick kit, manufactured by Qiagen may be used to extract cell-free DNA from plasma or serum.

The sample may be processed before the method is carried out, for example DNA purification may be carried out following the extraction procedure. The DNA in the sample may be cleaved either physically or chemically (e.g. using a suitable enzyme). Processing of the sample may involve one or more of: filtration, distillation, centrifugation, extraction, concentration, dilution, purification, inactivation of interfering components, addition of reagents, and the like.

To analyze methylation status according to this aspect of the present invention, the DNA may be treated with bisulfite which converts cytosine residues to uracil (which are converted to thymidine following PCR), but leaves 5-methylcytosine residues unaffected. Thus, bisulfite treatment introduces specific changes in the DNA sequence that depend on the methylation status of individual cytosine residues, yielding single- nucleotide resolution information about the methylation status of a segment of DNA.

During the bisulfite reaction, care should be taken to minimize DNA degradation, such as cycling the incubation temperature.

Bisulfite sequencing relies on the conversion of every single unmethylated cytosine residue to uracil. If conversion is incomplete, the subsequent analysis will incorrectly interpret the unconverted unmethylated cytosines as methylated cytosines, resulting in false positive results for methylation. Only cytosines in single-stranded DNA are susceptible to attack by bisulfite, therefore denaturation of the DNA undergoing analysis is critical. It is important to ensure that reaction parameters such as temperature and salt concentration are suitable to maintain the DNA in a single-stranded conformation and allow for complete conversion.

According to a particular embodiment, an oxidative bisulfite reaction is performed. 5-methylcytosine and 5-hydroxymethylcytosine both read as a C in bisulfite sequencing. Oxidative bisulfite reaction allows for the discrimination between 5-methylcytosine and 5-hydroxymethylcytosine at single base resolution. The method employs a specific chemical oxidation of 5-hydroxymethylcytosine to 5-formylcytosine, which subsequently converts to uracil during bisulfite treatment. The only base that then reads as a C is 5-methylcytosine, giving a map of the true methylation status in the DNA sample. Levels of 5-hydroxymethylcytosine can also be quantified by measuring the difference between bisulfite and oxidative bisulfite sequencing.

Optionally, the bisulfite-treated DNA molecules are subjected to an amplification reaction prior to, or concomitant with, analysis of the methylation pattern.

As used herein, the term "amplification" refers to a process that increases the representation of a population of specific nucleic acid sequences in a sample by producing multiple (i.e., at least 2) copies of the desired sequences. Methods for nucleic acid amplification are known in the art and include, but are not limited to, polymerase chain reaction (PCR) and ligase chain reaction (LCR). In a typical PCR amplification reaction, a nucleic acid sequence of interest is often amplified at least fifty thousand fold in amount over its amount in the starting sample. A "copy" or "amplicon" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable but not complementary to the template), and/or sequence errors that occur during amplification.

A typical amplification reaction is carried out by contacting a forward and reverse primer (a primer pair) to the sample DNA together with any additional amplification reaction reagents under conditions which allow amplification of the target sequence. The oligonucleotide amplification primers typically flank the target sequence - (i.e. the sequence comprising the at least one, two, three, four or five methylation sites (per single strand).

The terms "forward primer" and "forward amplification primer" are used herein interchangeably, and refer to a primer that hybridizes (or anneals) to the target (template strand). The terms "reverse primer" and "reverse amplification primer" are used herein interchangeably, and refer to a primer that hybridizes (or anneals) to the complementary target strand. The forward primer hybridizes with the target sequence 5' with respect to the reverse primer.

The term "amplification conditions", as used herein, refers to conditions that promote annealing and/or extension of primer sequences. Such conditions are well-known in the art and depend on the amplification method selected. Thus, for example, in a PCR reaction, amplification conditions generally comprise thermal cycling, i.e., cycling of the reaction mixture between two or more temperatures. In isothermal amplification reactions, amplification occurs without thermal cycling although an initial temperature increase may be required to initiate the reaction. Amplification conditions encompass all reaction conditions including, but not limited to, temperature and temperature cycling, buffer, salt, ionic strength, and pH, and the like.

As used herein, the term "amplification reaction reagents", refers to reagents used in nucleic acid amplification reactions and may include, but are not limited to, buffers, reagents, enzymes having reverse transcriptase and/or polymerase activity or exonuclease activity, enzyme cofactors such as magnesium or manganese, salts, nicotinamide adenine dinuclease (NAD) and deoxynucleoside triphosphates (dNTPs), such as deoxyadenosine triphospate, deoxyguanosine triphosphate, deoxycytidine triphosphate and thymidine triphosphate. Amplification reaction reagents may readily be selected by one skilled in the art depending on the amplification method used.

As a result of bisulfite conversion, the sequences of complementary DNA strands become less similar, such that base pairing does not occur anymore and the DNA becomes single stranded.

Thus, following bisulfite treatment, two strands of non-complementary DNA are generated:
(i) a forward single-stranded DNA molecule of which demethylated cytosines of the single-stranded DNA molecules are converted to uracils and;
(ii) a reverse single-stranded DNA molecule of which demethylated cytosines of the single-stranded DNA molecules are converted to uracils.

In one embodiment, the present invention contemplates analyzing the methylation pattern of both the forward strand of the DNA molecule and the reverse strand of the DNA molecule. Accordingly, the present inventors contemplate use of strand-specific oligonucleotides (either primers or probes as further described herein below).

The two amplification reactions may be carried out concomitantly (e.g. in the same reaction vessel, at the same time) or consecutively.

The present inventors contemplate fractionating the DNA from the sample/specimen prior to performing an amplification reaction. In one embodiment, the amplification reaction is a digital droplet PCR reaction (ddPCR).

To fractionate the DNA sample/specimen, eemulsification techniques can be used so as to create large numbers of aqueous droplets that function as independent reaction chambers for the PCR reactions. For example, an aqueous specimen (e.g., 20 microliters) can be partitioned into droplets (e.g., 20,000 droplets of one nanoliter each) to allow an individual test for the target to be performed with each of the droplets.

Aqueous droplets can be suspended in oil to create a water-in-oil emulsion (W/O). The emulsion can be stabilized with a surfactant to reduce coalescence of droplets during heating, cooling, and transport, thereby enabling thermal cycling to be performed.

In an exemplary droplet-based digital assay, a specimen is partitioned into a set of droplets at a dilution that ensures that more than 40% of the droplets contain no more than one single-stranded DNA molecule per specimen fraction.

In an exemplary droplet-based digital assay, a specimen is partitioned into a set of droplets at a dilution that ensures that more than 50% of the droplets contain no more than one single-stranded DNA molecule per specimen fraction.

In an exemplary droplet-based digital assay, a specimen is partitioned into a set of droplets at a dilution that ensures that more than 60% of the droplets contain no more than one single-stranded DNA molecule per specimen fraction.

In an exemplary droplet-based digital assay, a specimen is partitioned into a set of droplets at a dilution that ensures that more than 70% of the droplets contain no more than one single-stranded DNA molecule per specimen fraction.

In an exemplary droplet-based digital assay, a specimen is partitioned into a set of droplets at a dilution that ensures that more than 80% of the droplets contain no more than one single-stranded DNA molecule per specimen fraction.

In an exemplary droplet-based digital assay, a specimen is partitioned into a set of droplets at a dilution that ensures that more than 90% of the droplets contain no more than one single-stranded DNA molecule per specimen fraction.

Once fractionation has taken place, the single-stranded DNA may then optionally be amplified.

Whether subjected to fractionation or not, the primers which are used in the amplification reaction may be methylation-independent primers or methylation-dependent primers. Methylation-independent primers flank the first and last of the methylation sites of the signature (but do not hybridize directly to the sites) and in a PCR reaction, are capable of generating an amplicon which comprises the methylation sites of the methylation signature.

The methylation-independent primers may comprise adaptor sequences which include barcode sequences. The adaptors may further comprise sequences which are necessary for attaching to a flow cell surface (P5 and P7 sites, for subsequent sequencing), a sequence which encodes for a promoter for an RNA polymerase and/or a restriction site. The barcode sequence may be used to identify a particular molecule, sample or library. The barcode sequence may be between 3-400 nucleotides, more preferably between 3-200 and even more preferably between 3-100 nucleotides. Thus, the barcode sequence may be 6 nucleotides, 7 nucleotides, 8, nucleotides, nine nucleotides or ten nucleotides. The barcode is typically 4-15 nucleotides.

When methylation independent primers are used to amplify the target sequences, the sequence of the target sequence may be uncovered using sequencing techniques known in the art - e.g. massively parallel DNA sequencing, sequencing-by-synthesis, sequencing-by-ligation, 454 pyrosequencing, cluster amplification, bridge amplification, and PCR amplification, although preferably, the method comprises a high throughput sequencing method. Typical methods include the sequencing technology and analytical instrumentation offered by Roche 454 Life Sciences^{™}, Branford, Conn., which is sometimes referred to herein as "454 technology" or "454 sequencing."; the sequencing technology and analytical instrumentation offered by Illumina, Inc, San Diego, Calif. (their Solexa Sequencing technology is sometimes referred to herein as the "Solexa method" or "Solexa technology"); or the sequencing technology and analytical instrumentation offered by ABI, Applied Biosystems, Indianapolis, Ind., which is sometimes referred to herein as the ABI-SOLiD^{™} platform or methodology.

Other known methods for sequencing include, for example, those described in: Sanger, F. et al., Proc. Natl. Acad. Sci. U.S.A. 75, 5463-5467 (1977); Maxam, A. M. & Gilbert, W. Proc Natl Acad Sci USA 74, 560-564 (1977); Ronaghi, M. et al., Science 281, 363, 365 (1998); Lysov, 1. et al., Dokl Akad Nauk SSSR 303, 1508-1511 (1988); Bains W. & Smith G. C. J. Theor Biol 135, 303-307 (1988); Drnanac, R. et al., Genomics 4, 114-128 (1989); Khrapko, K. R. et al., FEBS Lett 256.118-122 (1989); Pevzner P. A. J Biomol Struct Dyn 7, 63-73 (1989); and Southern, E. M. et al., Genomics 13, 1008-1017 (1992). Pyrophosphate-based sequencing reaction as described, e.g., in U.S. Patent Nos. 6,274,320, 6,258,568 and 6,210,891, may also be used.

The Illumina or Solexa sequencing is based on reversible dye-terminators. DNA molecules are typically attached to primers on a slide and amplified so that local clonal colonies are formed. Subsequently one type of nucleotide at a time may be added, and non-incorporated nucleotides are washed away. Subsequently, images of the fluorescently labeled nucleotides may be taken and the dye is chemically removed from the DNA, allowing a next cycle. The Applied Biosystems' SOLiD technology, employs sequencing by ligation. This method is based on the use of a pool of all possible oligonucleotides of a fixed length, which are labeled according to the sequenced position. Such oligonucleotides are annealed and ligated. Subsequently, the preferential ligation by DNA ligase for matching sequences typically results in a signal informative of the nucleotide at that position. Since the DNA is typically amplified by emulsion PCR, the resulting bead, each containing only copies of the same DNA molecule, can be deposited on a glass slide resulting in sequences of quantities and lengths comparable to Illumina sequencing. Another example of an envisaged sequencing method is pyrosequencing, in particular 454 pyrosequencing, e.g. based on the Roche 454 Genome Sequencer. This method amplifies DNA inside water droplets in an oil solution with each droplet containing a single DNA template attached to a single primer-coated bead that then forms a clonal colony. Pyrosequencing uses luciferase to generate light for detection of the individual nucleotides added to the nascent DNA, and the combined data are used to generate sequence read-outs. A further method is based on Helicos' Heliscope technology, wherein fragments are captured by polyT oligomers tethered to an array. At each sequencing cycle, polymerase and single fluorescently labeled nucleotides are added and the array is imaged. The fluorescent tag is subsequently removed and the cycle is repeated. Further examples of sequencing techniques encompassed within the methods of the present invention are sequencing by hybridization, sequencing by use of nanopores, microscopy-based sequencing techniques, microfluidic Sanger sequencing, or microchip-based sequencing methods. The present invention also envisages further developments of these techniques, e.g. further improvements of the accuracy of the sequence determination, or the time needed for the determination of the genomic sequence of an organism etc.

According to one embodiment, the sequencing method comprises deep sequencing.

As used herein, the term "deep sequencing" and variations thereof refers to the number of times a nucleotide is read during the sequencing process. Deep sequencing indicates that the coverage, or depth, of the process is many times larger than the length of the sequence under study.

It will be appreciated that any of the analytical methods described herein can be embodied in many forms. For example, it can be embodied on a tangible medium such as a computer for performing the method operations. It can be embodied on a computer readable medium, comprising computer readable instructions for carrying out the method operations. It can also be embodied in electronic device having digital computer capabilities arranged to run the computer program on the tangible medium or execute the instruction on a computer readable medium.

Computer programs implementing the analytical method of the present embodiments can commonly be distributed to users on a distribution medium such as, but not limited to, CD-ROMs or flash memory media. From the distribution medium, the computer programs can be copied to a hard disk or a similar intermediate storage medium. In some embodiments of the present invention, computer programs implementing the method of the present embodiments can be distributed to users by allowing the user to download the programs from a remote location, via a communication network, e.g., the internet. The computer programs can be run by loading the computer instructions either from their distribution medium or their intermediate storage medium into the execution memory of the computer, configuring the computer to act in accordance with the method of this invention. All these operations are well-known to those skilled in the art of computer systems.

As mentioned, the present invention also contemplates use of methylation-sensitive oligomers as probes. The probes can be added during the amplification reaction (e.g. in a ddPCR reaction) as further described herein below.

In one embodiment, the amplification reaction includes a single labeled oligonucleotide probe which hybridizes to one strand of the amplified double-stranded DNA which comprises the methylation site. Thus, altogether the amplification reaction may include two labeled olignonucleotide probes - one which hybridizes to one strand of the amplified double-stranded DNA which comprises the methylation site originating from the forward strand of the original DNA and one which hybridizes to one strand of the amplified double-stranded DNA which comprises the methylation site originating from the reverse strand of the original DNA.

According to a particular embodiment, determining the methylation status is carried out as follows:
The DNA is contacted with:
(i) a first probe that hybridizes to at least one methylation site of the amplified DNA; and
(ii) a second probe that hybridizes to at least one other methylation site of the amplified DNA, wherein the first probe and the second probe are labeled with non-identical detectable moieties, wherein the first probe and the second probe comprise a quenching moiety.

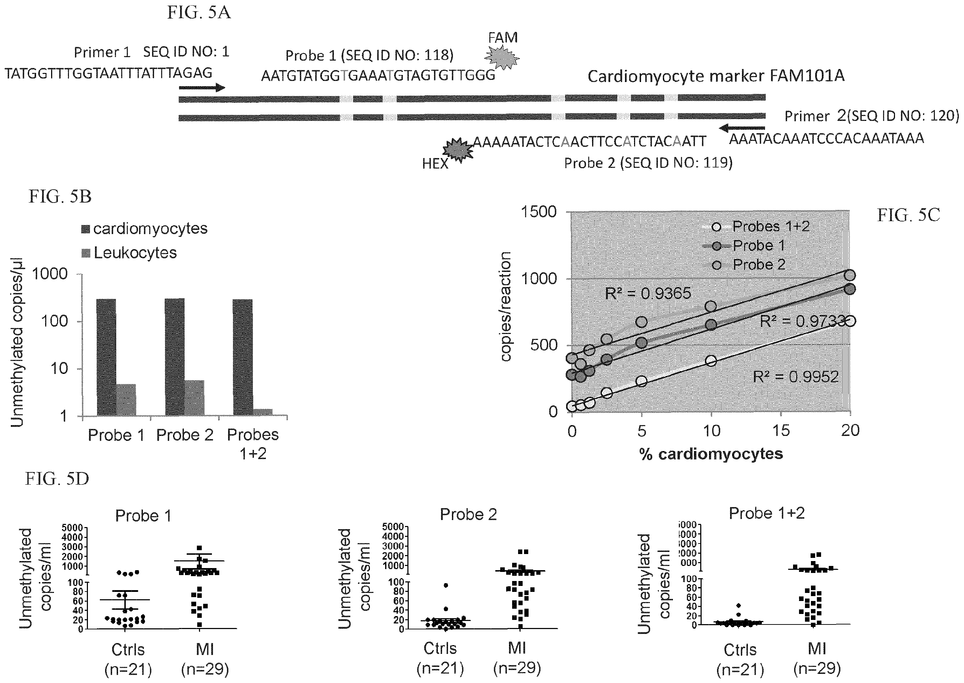

According to a particular embodiment, the first probe hybridizes to the forward strand of the amplified DNA and the second probe hybridizes to the reverse strand of the amplified DNA (see for example Figure 5A).

The contacting is effected under conditions that separate the quenching moiety from the first probe and the second probe to generate a non-quenched first probe and a non-quenched second probe. The conditions are those which are inductive for an amplification reaction - i.e. presence of a polymerase enzyme having 5' to 3' nuclease activity (e.g. Taqman polymerase), dNTPs and buffer etc.

Once sufficient amplification has occurred, the amount of non-quenched first probe and non-quenched second probe in a single droplet can be measured.

Preferably, when more than one probe is used in any of the amplification reactions described herein above, the probes are labeled with non-identical labels i.e. detectable moieties.

The oligonucleotides of the invention need not reflect the exact sequence of the target nucleic acid sequence (i.e. need not be fully complementary), but must be sufficiently complementary so as to hybridize to the target site under the particular experimental conditions. Accordingly, the sequence of the oligonucleotide typically has at least 70 % homology, preferably at least 80 %, 90 %, 95 %, 97 %, 99 % or 100 % homology, for example over a region of at least 13 or more contiguous nucleotides with the target sequence. The conditions are selected such that hybridization of the oligonucleotide to the target site is favored and hybridization to the non-target site is minimized.

Various considerations must be taken into account when selecting the stringency of the hybridization conditions. For example, the more closely the oligonucleotide (e.g. primer) reflects the target nucleic acid sequence, the higher the stringency of the assay conditions can be, although the stringency must not be too high so as to prevent hybridization of the oligonucleotides to the target sequence. Further, the lower the homology of the oligonucleotide to the target sequence, the lower the stringency of the assay conditions should be, although the stringency must not be too low to allow hybridization to non-specific nucleic acid sequences.

Oligonucleotides of the invention may be prepared by any of a variety of methods (see, for example, J. Sambrook et al., "Molecular Cloning: A Laboratory Manual", 1989, 2.sup.nd Ed., Cold Spring Harbour Laboratory Press: New York, N.Y.; "PCR Protocols: A Guide to Methods and Applications", 1990, M. A. Innis (Ed.), Academic Press: New York, N.Y.; P. Tijssen "Hybridization with Nucleic Acid Probes--Laboratory Techniques in Biochemistry and Molecular Biology (Parts I and II)", 1993, Elsevier Science; "PCR Strategies", 1995, M. A. Innis (Ed.), Academic Press: New York, N.Y.; and "Short Protocols in Molecular Biology", 2002, F. M. Ausubel (Ed.), 5.sup.th Ed., John Wiley & Sons: Secaucus, N.J.). For example, oligonucleotides may be prepared using any of a variety of chemical techniques well-known in the art, including, for example, chemical synthesis and polymerization based on a template as described, for example, in S. A. Narang et al., Meth. Enzymol. 1979, 68: 90-98; E. L. Brown et al., Meth. Enzymol. 1979, 68: 109-151; E. S. Belousov et al., Nucleic Acids Res. 1997, 25: 3440-3444; D. Guschin et al., Anal. Biochem. 1997, 250: 203-211; M. J. Blommers et al., Biochemistry, 1994, 33: 7886-7896; and K. Frenkel et al., Free Radic. Biol. Med. 1995, 19: 373-380; and U.S. Pat. No. 4,458,066.

For example, oligonucleotides may be prepared using an automated, solid-phase procedure based on the phosphoramidite approach. In such a method, each nucleotide is individually added to the 5'-end of the growing oligonucleotide chain, which is attached at the 3'-end to a solid support. The added nucleotides are in the form of trivalent 3'-phosphoramidites that are protected from polymerization by a dimethoxytriyl (or DMT) group at the 5'-position. After base-induced phosphoramidite coupling, mild oxidation to give a pentavalent phosphotriester intermediate and DMT removal provides a new site for oligonucleotide elongation. The oligonucleotides are then cleaved off the solid support, and the phosphodiester and exocyclic amino groups are deprotected with ammonium hydroxide. These syntheses may be performed on oligo synthesizers such as those commercially available from Perkin Elmer/Applied Biosystems, Inc. (Foster City, Calif.), DuPont (Wilmington, Del.) or Milligen (Bedford, Mass.). Alternatively, oligonucleotides can be custom made and ordered from a variety of commercial sources well-known in the art, including, for example, the Midland Certified Reagent Company (Midland, Tex.), ExpressGen, Inc. (Chicago, Ill.), Operon Technologies, Inc. (Huntsville, Ala.), and many others.

Purification of the oligonucleotides of the invention, where necessary or desirable, may be carried out by any of a variety of methods well-known in the art. Purification of oligonucleotides is typically performed either by native acrylamide gel electrophoresis, by anion-exchange HPLC as described, for example, by J. D. Pearson and F. E. Regnier (J. Chrom., 1983, 255: 137-149) or by reverse phase HPLC (G. D. McFarland and P. N. Borer, Nucleic Acids Res., 1979, 7: 1067-1080).

The sequence of oligonucleotides can be verified using any suitable sequencing method including, but not limited to, chemical degradation (A. M. Maxam and W. Gilbert, Methods of Enzymology, 1980, 65: 499-560), matrix-assisted laser desorption ionization time-of-flight (MALDI-TOF) mass spectrometry (U. Pieles et al., Nucleic Acids Res., 1993, 21: 3191-3196), mass spectrometry following a combination of alkaline phosphatase and exonuclease digestions (H. Wu and H. Aboleneen, Anal. Biochem., 2001, 290: 347-352), and the like.

In certain embodiments, the detection probes or amplification primers or both probes and primers are labeled with a detectable agent or moiety before being used in amplification/detection assays. In certain embodiments, the detection probes are labeled with a detectable agent. Preferably, a detectable agent is selected such that it generates a signal which can be measured and whose intensity is related (e.g., proportional) to the amount of amplification products in the sample being analyzed.

The association between the oligonucleotide and detectable agent can be covalent or noncovalent. Labeled detection probes can be prepared by incorporation of or conjugation to a detectable moiety. Labels can be attached directly to the nucleic acid sequence or indirectly (e.g., through a linker). Linkers or spacer arms of various lengths are known in the art and are commercially available, and can be selected to reduce steric hindrance, or to confer other useful or desired properties to the resulting labeled molecules (see, for example, E. S. Mansfield et al., Mol. Cell. Probes, 1995, 9: 145-156).

Methods for labeling nucleic acid molecules are well-known in the art. For a review of labeling protocols, label detection techniques, and recent developments in the field, see, for example, L. J. Kricka, Ann. Clin. Biochem. 2002, 39: 114-129; R. P. van Gijlswijk et al., Expert Rev. Mol. Diagn. 2001, 1: 81-91; and S. Joos et al., J. Biotechnol. 1994, 35: 135-153. Standard nucleic acid labeling methods include: incorporation of radioactive agents, direct attachments of fluorescent dyes (L. M. Smith et al., Nucl. Acids Res., 1985, 13: 2399-2412) or of enzymes (B. A. Connoly and O. Rider, Nucl. Acids. Res., 1985, 13: 4485-4502); chemical modifications of nucleic acid molecules making them detectable immunochemically or by other affinity reactions (T. R. Broker et al., Nucl. Acids Res. 1978, 5: 363-384; E. A. Bayer et al., Methods of Biochem. Analysis, 1980, 26: 1-45; R. Langer et al., Proc. Natl. Acad. Sci. USA, 1981, 78: 6633-6637; R. W. Richardson et al., Nucl. Acids Res. 1983, 11: 6167-6184; D. J. Brigati et al., Virol. 1983, 126: 32-50; P. Tchen et al., Proc. Natl. Acad. Sci. USA, 1984, 81: 3466-3470; J. E. Landegent et al., Exp. Cell Res. 1984, 15: 61-72; and A. H. Hopman et al., Exp. Cell Res. 1987, 169: 357-368); and enzyme-mediated labeling methods, such as random priming, nick translation, PCR and tailing with terminal transferase (for a review on enzymatic labeling, see, for example, J. Temsamani and S. Agrawal, Mol. Biotechnol. 1996, 5: 223-232). More recently developed nucleic acid labeling systems include, but are not limited to: ULS (Universal Linkage System), which is based on the reaction of mono-reactive cisplatin derivatives with the N7 position of guanine moieties in DNA (R. J. Heetebrij et al., Cytogenet. Cell. Genet. 1999, 87: 47-52), psoralen-biotin, which intercalates into nucleic acids and upon UV irradiation becomes covalently bonded to the nucleotide bases (C. Levenson et al., Methods Enzymol. 1990, 184: 577-583; and C. Pfannschmidt et al., Nucleic Acids Res. 1996, 24: 1702-1709), photoreactive azido derivatives (C. Neves et al., Bioconjugate Chem. 2000, 11: 51-55), and DNA alkylating agents (M. G. Sebestyen et al., Nat. Biotechnol. 1998, 16: 568-576).

If the methylation sites are close enough together on the DNA, it is conceivable that the probes of this aspect of the present invention hybridize to more than one methylation site, for example, two, three, or even four.

The sequence of the first and/or second probe may be selected such that it binds to the amplified DNA when the methylation site of the double-stranded DNA molecule is non-methylated.

Alternatively, the sequence of the first and/or second probe may be selected such that it binds to the amplified DNA when the methylation site of the double-stranded DNA molecule is methylated.

In certain embodiments, the inventive detection probes are fluorescently labeled. Numerous known fluorescent labeling moieties of a wide variety of chemical structures and physical characteristics are suitable for use in the practice of this invention. Suitable fluorescent dyes include, but are not limited to, fluorescein and fluorescein dyes (e.g., fluorescein isothiocyanine or FITC, naphthofluorescein, 4',5'-dichloro-2',7'-dimethoxy-fluorescein, 6 carboxyfluorescein or FAM), carbocyanine, merocyanine, styryl dyes, oxonol dyes, phycoerythrin, erythrosin, eosin, rhodamine dyes (e.g., carboxytetramethylrhodamine or TAMRA, carboxyrhodamine 6G, carboxy-X-rhodamine (ROX), lissamine rhodamine B, rhodamine 6G, rhodamine Green, rhodamine Red, tetramethylrhodamine or TMR), coumarin and coumarin dyes (e.g., methoxycoumarin, dialkylaminocoumarin, hydroxycoumarin and aminomethylcoumarin or AMCA), Oregon Green Dyes (e.g., Oregon Green 488, Oregon Green 500, Oregon Green 514), Texas Red, Texas Red-X, Spectrum Red^{™}, Spectrum Green^{™}, cyanine dyes (e.g., Cy-3^{™}, Cy-5^{™}, Cy-3.5^{™}, Cy-5.5^{™}, Alexa Fluor dyes (e.g., Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), BODIPY dyes (e.g., BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), IRDyes (e.g., IRD40, IRD 700, IRD 800), and the like. For more examples of suitable fluorescent dyes and methods for linking or incorporating fluorescent dyes to nucleic acid molecules see, for example, "The Handbook of Fluorescent Probes and Research Products", 9th Ed., Molecular Probes, Inc., Eugene, Oreg. Fluorescent dyes as well as labeling kits are commercially available from, for example, Amersham Biosciences, Inc. (Piscataway, N.J.), Molecular Probes Inc. (Eugene, Oreg.), and New England Biolabs Inc. (Beverly, Mass.).Another contemplated method of analyzing the methylation status of the sequences is by analysis of the DNA following exposure to methylation-sensitive restriction enzymes - see for example US Application Nos. 20130084571 and 20120003634, the contents of which are incorporated herein.

Exemplary probes for identifying cardiac cells are set forth in SEQ ID NOs: 118 and 119.

Exemplary probes for identifying liver cells are set forth in SEQ ID NOs: 178, 179 and 182.

In one embodiment, the amplification reaction uses at least two TaqMan^{™} probes, one which hybridizes to the first strand of the amplified DNA and one which hybridizes to the second strand of the amplified DNA.

TaqMan^{™} probes comprise a detectable moiety (e.g. fluorophore) covalently attached to the 5'-end of the oligonucleotide probe and a quencher at the 3'-end. Several different fluorophores (e.g. 6-carboxyfluorescein, acronym: FAM, or tetrachlorofluorescein, acronym: TET) and quenchers (e.g. tetramethylrhodamine, acronym: TAMRA) are available. The quencher molecule quenches the fluorescence emitted by the fluorophore when excited by the cycler's light source via FRET (Förster Resonance Energy Transfer).^{[6]} As long as the fluorophore and the quencher are in proximity, quenching inhibits any fluorescence signals.

TaqMan^{™} probes are designed such that they anneal within a DNA region amplified by a specific set of primers. As the Taq polymerase extends the primer and synthesizes the nascent strand, the 5' to 3' exonuclease activity of the Taq polymerase degrades the probe that has annealed to the template. Degradation of the probe releases the detectable moiety from it and breaks the close proximity to the quencher, thus relieving the quenching effect and allowing for detection of the detectable moiety (e.g. it allow for fluorescence of the fluorophore). Hence, the amount of detectable moiety is directly proportional to the amount of DNA template present in the PCR.

### Kits

Any of the components described herein may be comprised in a kit. In a non-limiting example the kit comprises at least two oligonucleotides which are capable of amplifying a DNA molecule having a nucleic acid sequence no longer than 300 base pairs, wherein the nucleic acid sequence comprises at least two methylation sites which are differentially methylated in a first cell of interest with respect to a second cell which is non-identical to the first cell of interest, wherein the nucleic acid sequence is comprised in a sequence as set forth in any one of SEQ ID Nos: 2-117 or 121-177.

Detectable moieties, quenching moieties and probes have been described herein above.

Additional components that may be included in any of the above described kits include at least one of the following components: a droplet forming oil, bisulfite (and other reagents necessary for the bisulfite reaction), reagents for purification of DNA, MgCl₂. The kit may also comprise reaction components for sequencing the amplified or non-amplified sequences.

The kits may also comprise DNA sequences which serve as controls. Thus, for example, the kit may comprise a DNA having the same sequence as the amplified sequence derived from a healthy subject (to serve as a negative control) and/or a DNA having the same sequence as the amplified sequence derived from a subject known to have the disease which is being investigated (to serve as a positive control).

In addition, the kits may comprise known quantities of DNA such that calibration and quantification of the test DNA may be carried out.

The containers of the kits will generally include at least one vial, test tube, flask, bottle, syringe or other containers, into which a component may be placed, and preferably, suitably aliquoted. Where there is more than one component in the kit, the kit also will generally contain a second, third or other additional container into which the additional components may be separately placed. However, various combinations of components may be comprised in a container.

When the components of the kit are provided in one or more liquid solutions, the liquid solution can be an aqueous solution. However, the components of the kit may be provided as dried powder(s). When reagents and/or components are provided as a dry powder, the powder can be reconstituted by the addition of a suitable solvent.

A kit will preferably include instructions for employing, the kit components as well the use of any other reagent not included in the kit. Instructions may include variations that can be implemented.

### Diagnostics

It will be appreciated that analysis of the methylation status using the target sequences described herein allows for the accurate determination of cellular/tissue source of a DNA molecule, even when the majority of the DNA of the sample is derived from a different cellular source. The present inventors have shown that they are able to determine the cellular source of a particular DNA even when its contribution to the total amount of DNA in the population is less than 1:1000, less than 1:5,000, 1:10,000 or even 1:100,000.

Pathological and disease conditions that involve cell death cause the release of degraded DNA from dying cells into body fluids (blood, plasma, urine, cerebrospinal fluid). Thus, the methods described herein may be used to analyze the amount of cell death of a particular cell population in those body fluids. The amount of cell death of a particular cell population can then be used to diagnose a particular pathological state (e.g. disease) or condition (e.g. trauma).

It will be appreciated that death of a particular cell type may be associated with a pathological state - e.g. disease or trauma.

The monitoring of the death of a particular cell type may also be used for monitoring the efficiency of a therapeutic regime expected to effect cell death of a specific cell type.

The determination of death of a specific cell type may also be used in the clinical or scientific study of various mechanism of healthy or diseased subjects.

Thus, for example measurement of pancreatic beta cell death is important in cases of diabetes, hyperinsulinism and islet cell tumors, and in order to monitor beta cell survival after islet transplantation, determining the efficacy of various treatment regimes used to protect beta cells from death, and determining the efficacy of treatments aimed at causing islet cell death in islet cell tumors. Similarly, the method allows the identification and quantification of DNA derived from dead kidney cells (indicative of kidney failure), dead neurons (indicative of traumatic brain injury, amyotrophic lateral sclerosis (ALS), stroke, Alzheimer's disease, Parkinson's disease or brain tumors, with or without treatment); dead pancreatic acinar cells (indicative of pancreatic cancer or pancreatitis); dead lung cells (indicative of lung pathologies including lung cancer); dead adipocytes (indicative of altered fat turnover), dead hepatocytes (indicative of liver failure, liver toxicity or liver cancer) dead cardiomyocytes (indicative of cardiac disease, or graft failure in the case of cardiac transplantation), dead skeletal muscle cells (indicative of muscle injury and myopathies), dead oligodendrocytes (indicative of relapsing multiple sclerosis, white matter damage in amyotrophic lateral sclerosis, or glioblastoma), dead colon cells is indicative of colorectal cancer.

As used herein, the term "diagnosing" refers to determining the presence of a disease, classifying a disease, determining a severity of the disease (grade or stage), monitoring disease progression and response to therapy, forecasting an outcome of the disease and/or prospects of recovery.

The method comprises quantifying the amount of cell-free DNA which is comprised in a fluid sample (e.g. a blood sample or serum sample) of the subject which is derived from a cell type or tissue. When the amount of cell free DNA derived from the cell type or tissue is above a predetermined level, it is indicative that there is a predetermined level of cell death. When the level of cell death is above a predetermined level, it is indicative that the subject has the disease or pathological state. Determining the predetermined level may be carried out by analyzing the amount of cell-free DNA present in a sample derived from a subject known not to have the disease/pathological state. If the level of the cell-free DNA derived from a cell type or tissue associated with the disease in the test sample is statistically significantly higher (e.g. at least two fold, at least three fold, or at least 4 fold) than the level of cell-free DNA derived from the same cell type or tissue in the sample obtained from the healthy (non-diseased subject), it is indicative that the subject has the disease. Alternatively, or additionally, determining the predetermined level may be carried out by analyzing the amount of cell-free DNA present in a sample derived from a subject known to have the disease. If the level of the cell-free DNA derived from a cell type or tissue associated with the disease in the test sample is statistically significantly similar to the level of the cell-free DNA derived from a cell type of tissue associated with the disease in the sample obtained from the diseased subject, it is indicative that the subject has the disease.

The severity of disease may be determined by quantifying the amount of DNA molecules having the specific methylation pattern of a cell population associated with the disease. Quantifying the amount of DNA molecules having the specific methylation pattern of a target tissue may be achieved using a calibration curve produced by using known and varying numbers of cells from the target tissue.

According to one embodiment, the method comprises determining the ratio of the amount of cell free DNA derived from a cell of interest in the sample: amount of overall cell free DNA.

According to still another embodiment, the method comprises determining the ratio of the amount of cell free DNA derived from a cell of interest in the sample: amount of cell free DNA derived from a second cell of interest.

The methods described herein may also be used to determine the efficacy of a therapeutic agent or treatment, wherein when the amount of DNA associated with a cell population associated with the disease is decreased following administration of the therapeutic agent, it is indicative that the agent or treatment is therapeutic.

According to another aspect of the present invention there is provided a method of classifying (i.e. determining the severity of) a disease or disorder associated with tissue damage of a subject, the method comprising analyzing cell-free DNA derived from the tissue in a fluid sample of the subject, wherein the amount of the cell-free DNA is indicative of a classification of the disease or disorder.

In one embodiment, the disease is classified as being mild, moderate or severe according to the level of identified cell-free DNA.

Particular contemplated diseases include but are not limited to sepsis, lupus or HIV.

The method can also be used to diagnose a subject with the disease (sepsis, lupus or HIV).

According to some embodiments of the invention, screening of the subject for a specific disease is followed by substantiation of the screen results using gold standard methods.

The method can also be used to predict prognosis of the subject with the disease.

According to some embodiments of the invention, the method further comprising informing the subject of the predicted disease and/or the predicted prognosis of the subject.

As used herein the phrase "informing the subject" refers to advising the subject that based on the cfDNA levels, the subject should seek a suitable treatment regimen.

Once the cfDNA level is determined, the results can be recorded in the subject's medical file, which may assist in selecting a treatment regimen and/or determining prognosis of the subject.

According to some embodiments of the invention, the method further comprising recording the cf DNA levels of the subject in the subject's medical file.

As mentioned, the prediction can be used to select the treatment regimen of a subject and thereby treat the subject in need thereof.

As used herein the term "about" refers to ± 10 %

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is understood that any Sequence Identification Number (SEQ ID NO) disclosed in the instant application can refer to either a DNA sequence or a RNA sequence, depending on the context where that SEQ ID NO is mentioned, even if that SEQ ID NO is expressed only in a DNA sequence format or a RNA sequence format. For example, SEQ ID NO: XXX is expressed in a DNA sequence format (e.g., reciting T for thymine), but it can refer to either a DNA sequence that corresponds to an XXX nucleic acid sequence, or the RNA sequence of an RNA molecule nucleic acid sequence. Similarly, though some sequences are expressed in a RNA sequence format (e.g., reciting U for uracil), depending on the actual type of molecule being described, it can refer to either the sequence of a RNA molecule comprising a dsRNA, or the sequence of a DNA molecule that corresponds to the RNA sequence shown. In any event, both DNA and RNA molecules having the sequences disclosed with any substitutes are envisioned.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXAMPLE 1

### Analyzing methylation patterns of cardiac markers

### MATERIALS AND METHODS

***Clinical samples:*** Cardiac biomarkers used were troponin T and CPK.

***Identification of cardiac methylation markers:*** Tissue-specific DNA methylation markers were selected after a comparison of publically available DNA methylation datasets generated by whole-genome bisulfite sequencing (Roadmap Epigenomics). The fragment of FAM101A used as a cariomyocyte-specific marker is located in chromosome 12, coordinates 124692462-124692551.

***cfDNA analysis:*** Blood samples were collected in EDTA tubes, and centrifuged within 2 hours to separate plasma from peripheral blood cells: first at 1500g for 10 min, and then at 3000g for 10 min to remove any remaining cells. Plasma was then stored at -80°C.

cfDNA was extracted using the QIAsymphony SP instrument and its dedicated QIAsymphony Circulating DNA Kit (Qiagen) according to the manufacturer's instructions. DNA concentration was measured using the Qubit^{™} dsDNA HS Assay Kit.

cfDNA was treated with bisulfite using a kit (Zymo Research), and PCR amplified with primers specific for bisulfite-treated DNA but independent of methylation status at the monitored CpG sites. Primers were bar-coded, allowing the mixing of samples from different individuals when sequencing PCR products using MiSeq or NextSeq (Illumina). Sequenced reads were separated by barcode, aligned to the target sequence, and analyzed using custom scripts written and implemented in R. Reads were quality filtered based on Illumina quality scores, and identified by having at least 80% similarity to target sequences and containing all the expected CpGs in the sequence. CpGs were considered methylated if "CG" was read and were considered unmethylated if "TG" was read.

***Digital Droplet PCR:*** A procedure was established for digital droplet PCR, in which bisulfite-treated cfDNA is amplified using a methylation-sensitive Taqman^{™} probe.

The limited length of probes (up to 30 bp) dictated that they could cover only 2 or 3 informative CpG sites in the FAM101A locus, predicting a relatively high frequency of "noise" (positive droplets) in DNA from non-cardiac tissue. In the sequencing-based assay, this problem was addressed by documenting the methylation status of multiple adjacent cytosines (Figures 1A-E), which greatly increased specificity.

To implement this concept in the ddPCR platform,two Taqman^{™} probes were designed, each recognizing lack of methylation in a different cluster of cytosines (one containing 2 CpG sites and one containing 3 CpG sites) within the same amplified 100bp fragment from the FAM101A locus (Figure 5A). Each probe was labeled with a different fluorophore, such that droplets could be identified in which both probes found a target. Such droplets would be interpreted as containing a FAM101A cfDNA fragment in which all 5 targeted cytosines were demethylated. This would provide ddPCR with the improved specificity afforded by interrogating multiple cytosines on the same DNA molecule.

For the analysis of 5 cytosines, located adjacent to the FAM101A locus, the following primers were used: 5'-TATGGTTTGGTAATTTATTTAGAG-3' (SEQ ID NO: 1; forward) and 5'-AAATACAAATCCCACAAATAAA-3' (SEQ ID NO: 120; reverse) in combination with probes that detected lack of methylation on 3 and 2 cytosines respectively: 5'-AATGTATGGTGAAATGTAGTGTTGGG -3' (SEQ ID NO: 118; FAM-forward probe) and 5'- AAAAATACTCAACTTCCATCTACAATT -3' (SEQ ID NO: 119, HEX-reverse probe).

Assay design is shown in Figure 5A. Each 20-µL volume reaction mix consisted of ddPCR^{™} Supermix for Probes (No dUTP) (Bio-Rad), 900nM primer, 250 nM probe, and 2 µL of sample. The mixture and droplet generation oil were loaded onto a droplet generator (Bio-Rad). Droplets were transferred to a 96-well PCR plate and sealed. The PCR was run on a thermal cycler as follows: 10 minutes of activation at 95 °C, 47 cycles of a 2 step amplification protocol (30 s at 94 °C denaturation and 60 s at 53.7 °C), and a 10-minute inactivation step at 98°C. The PCR plate was transferred to a QX100Droplet Reader (Bio-Rad), and products were analyzed with QuantaSoft (Bio-Rad) analysis software. Discrimination between droplets that contained the target (positives) and those which did not (negatives) was achieved by applying a fluorescence amplitude threshold based on the amplitude of reads from the negative template control.

### RESULTS

### Identification of cardiomyocyte methylation markers

To define genomic loci that are methylated in a cardiac-specific manner, the methylomes of human heart chambers (right atrium, left and right ventricle) were compared with the methylomes of 23 other human tissues, all publicly available¹². Several differentially methylated loci were identified and a cluster of cytosines adjacent to the FAM101A locus was selected for further analysis (Figures 1A and B). PCR was used to amplify a 90bp fragment around this cluster after bisulfite conversion of unmethylated cytosines, and the PCR product was sequenced to determine the methylation status of all 6 cytosines in the cluster. In purified cardiomyocyte DNA, 89% of the molecules were fully unmethylated, while in non-cardiac tissue <0.2% of molecules were unmethylated; specifically in leukocytes (the main contributor to cfDNA), <0.006% of molecules were unmethylated (Figures 1C and 6A-C). Thus, interrogating all CpGs simultaneously, the ratio of demethylated molecules in heart:blood DNA was 89:0.006 giving a signal to noise ratio of 15,000.

To determine the linearity and sensitivity of the assay, leukocyte DNA was spiked with increasing amounts of cardiac DNA. The fraction of cardiac DNA in the mixture was assessed using PCR amplification and massively parallel sequencing. The assay was able to correctly determine the fraction of cardiac DNA, even when it was only 0.5% of the DNA in the mixture (Figure 1D).

Following bisulfite treatment, DNA becomes single stranded. Therefore, each strand can be considered an independent biomarker. To test this idea, the present inventors designed primers against the antisense strand of FAM101A post-bisulfite conversion. As expected, the sense and antisense templates showed a similar sensitivity and specificity (Figures 1B-E and 6A-C). It was reasoned that by testing both strands in a given sample, both sensitivity and specificity of the assay will increase. For this reason further analysis of clinical samples was performed using both sense and antisense specific primer sets.

### Plasma levels of cardiomyocyte DNA in healthy individuals

The sense and antisense FAM101A markers were used to assess the concentration of cardiac cfDNA in the plasma of donors. cfDNA was extracted from plasma and treated with bisulfite. PCR and sequencing were performed, typically using material from 0.5ml of plasma. The fraction of PCR products carrying the cardiac-specific methylation pattern was multiplied by the total concentration of cfDNA, to obtain an estimation of cardiac cfDNA content in plasma.

Healthy adult plasma from 83 healthy donors was tested and zero copies of cardiac cfDNA were detected in 73 of them (Figure 2A). In ten individuals, 1-20 copies/ml cardiac cfDNA was found. This low level of a signal likely reflects the low rate of cardiomyocyte death in healthy adults¹³. The mean plus 2 standard deviations of the control group was 10 copies/ml, and this was thus defined as the cutoff level for a positive signal.

***Plasma levels of cardiomyocyte DNA after myocardial infarction:*** As a positive control where high levels of cardiac cfDNA are expected, plasma from donors with myocardial infarction (MI) were used. Samples from individuals that presented with chest pain, before and after they underwent angioplasty were used. The levels of cardiac cfDNA as well as troponin and CPK were assessed. MI patients showed dramatically higher levels of cardiac cfDNA than healthy controls (Figure 2A and Figures 7A-F and 8A-B). To assess assay performance in discriminating healthy from MI plasma a Receiver Operator Characteristic (ROC) curve was plotted. The area under the curve (AUC) was 0.9345, indicating high sensitivity and specificity (Figure 2B). The present inventors also compared cardiac cfDNA to standard cardiac damage markers CPK and troponin. Compared with healthy controls, cardiac cfDNA was significantly higher in MI patients that had CPK just above normal (<200), and was even higher in patients with high CPK (>200) (Figure 2C). Similarly, cardiac cfDNA was higher than normal in plasma samples that had either low or high levels of troponin (Figure 2D and Figures 7A-F). Among the 6 samples that had troponin levels above baseline but <0.03, there was no more cfDNA than in healthy controls (Figure 2D).

A comparison of troponin levels to cardiac cfDNA in 57 samples from MI patients yielded Spearman correlation value of 0.7975 and p<0.0001 (Figure 2E). When plotting cardiac cfDNA vs troponin and marking on each axis the threshold of a positive signal, it was found that 79% of the MI samples were positive for both troponin and cardiac cfDNA, and 7% were negative for both. 11% were positive only for troponin, and 4% were positive only for cardiac cfDNA (Figure 2F). Importantly, total levels of cfDNA in MI did not correlate with troponin or CPK, nor with the percentage of cardiac cfDNA (Figures 7A-F). This reflects that fact that total cfDNA integrates all recent cell death events, including contributions from tissues that mask the cardiac signal. Thus, it is essential to calculate the specific contribution of the heart to cfDNA in order to assess cardiac damage. The sense and antisense markers correlated well in the MI plasma samples (Figures 7A-F).

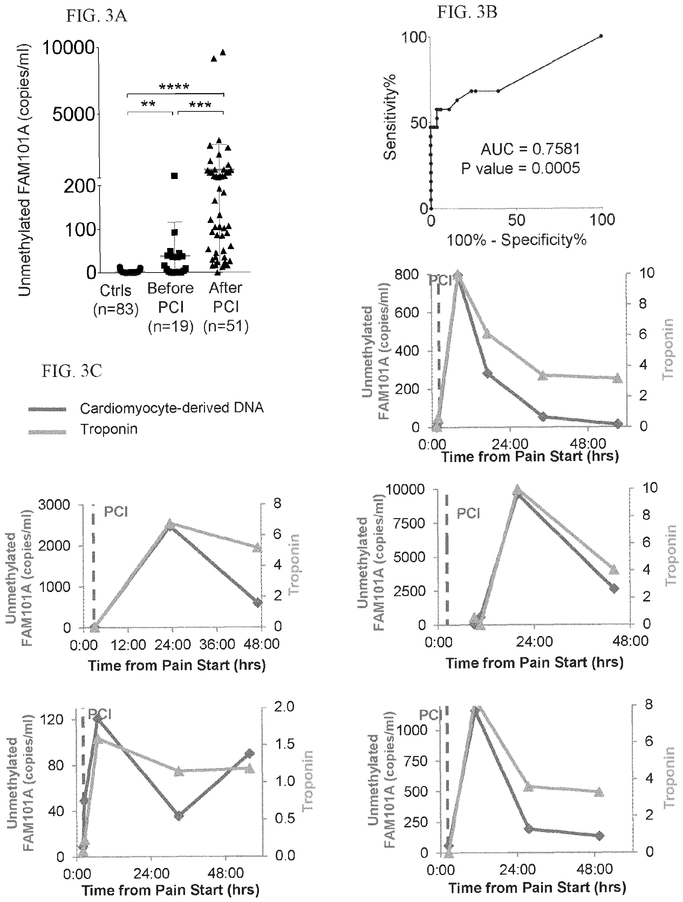

Finally, the present inventors examined the dynamics of cardiac cfDNA before and afer angioplasty (Percutaneous Coronary Intervention, PCI). PCI causes the release of trapped cardiac material into blood, hence increased levels of troponin post PCI are typical of successful reperfusion. Cardiac cfDNA levels increased dramatically in most patients after PCI (Figure 3A and Figures 8A-B), further supporting authenticity of the signal. A more detailed time course on a smaller group of patients revealed that cardiac cfDNA levels rose quickly after PCI and returned to baseline after 1-2 days, showing similar kinetics to troponin and CPK (Figure 3B and Figures 8A-B). Importantly, the cardiac cfDNA signal was sufficient to distinguish people with MI prior to intervention (0-2 hours after onset of chest pain) from healthy individuals (AUC=0.7616, p=0.0044, Figure 3C).

It can be concluded that measurements of cardiac cfDNA captures cardiomyocyte cell death associated with myocardial infarction, and that the cardiac cfDNA assay can in principle identify MI before intervention.

### Cardiomyocyte cfDNA in patients with sepsis

Some septic patients have elevated levels of troponin and CPK¹⁴, although they do not show clinical evidence of cardiac damage ^{15, 16}. The biological significance of this observation is disputed, since high troponin could represent either cardiomyocyte death, or alternatively transient stress absent of cell death. Since renal dysfunction is common in sepsis, the elevation in circulating troponin may also result from slower clearance, rather than faster release of troponin¹⁷. Since cfDNA is a stronger marker of cell death and is cleared by the liver¹⁸, it was reasoned that measurements of cardiac cfDNA can be informative in this setting.

The present inventors determined the levels of cardiac cfDNA in a cohort of 100 patients with sepsis, for which 201 plasma samples were available. Cardiac cfDNA was assessed blindly, and values were correlated to other biomarkers and to clinical parameters.

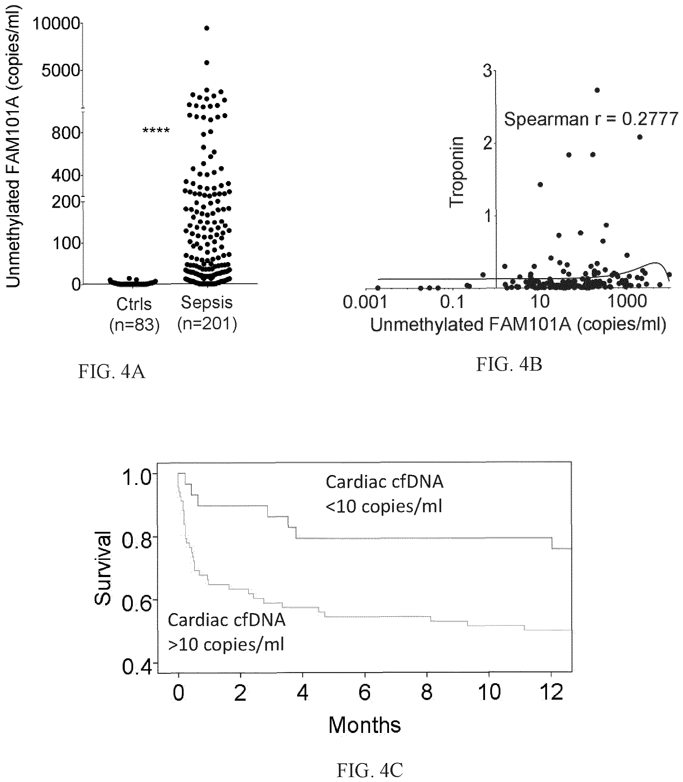

Septic patients had high levels of total cfDNA, reflective of broad tissue damage (Figures 9A-C), as reported¹⁹. Strikingly, many patients had high levels of cardiac cfDNA, similar in magnitude to the acute setting of MI (Figure 4A). These findings argue strongly that in many septic patients, massive cardiomyocyte death occurs. The sense and antisense markers of FAM101A correlated well, supporting specificity of the signal (Figures 9A-C). Cardiac cfDNA and troponin levels did not correlate in the sepsis, unlike the situation in MI (Figure 4B). This is not surprising, given the chronic nature of tissue damage in sepsis, which is expected to involve a major contribution of clearance rates on the actual measurements of biomarkers. A dramatic elevation of cardiac cfDNA was seen also in septic patients with normal renal function (data not shown), supporting the idea that cardiac cfDNA reflects cell death and not altered clearance rate.

The present inventors attempted to correlate the levels of cardiac cfDNA with clinical parameters recorded for the sepsis patients. The presence of cardiac cfDNA was strongly correlated with short-term mortality (Figure 4C). When excluding cases with sepsis in the background of advanced cancer, patients with cardiac cfDNA were 4 times more likely to die within 90 days of hospitalization than patients with no cardiac cfDNA. The correlation was stronger than the correlation between troponin and mortality or between total cfDNA and mortality, but weaker than the correlation between age and mortality. These findings indicate that cardiac function is a central determinant of patient survival under sepsis, and that cardiac cfDNA can be used as a prognostic biomarker in sepsis.

### A modified digital droplet PCR procedure for measurement of cardiac cfDNA

In order to translate analysis of cfDNA to a simpler and faster PCR format, the present inventors established a procedure using digital droplet PCR (ddPCR) to accurately count the number of molecules carrying the cardiac methylation signature at the FAM101A locus. They designed the assay to simultaneously interrogate 5 CpGs in the locus using two fluorescent probes, each capturing distinct 2 or 3 unmethylated cytosines (Figure 5A), leveraging the increased specificity attributed to regional methylation status⁹.

ddPCR analysis of cardiomyocyte and leukocyte DNA revealed that each probe alone was able to discriminate between DNA from the two sources, with a signal to noise ratio of 50 to 58. However, when only droplets positive for both probes were scored, the cardiomyocyte: leukocyte signal ratio increased to 258, affording a 5 fold increase in specificity (Figure 5B). ddPCR on cardiac DNA spiked into leukocyte DNA gave a signal that increased linearly with the amount of cardiac DNA; scoring only dual-labeled probes gave a lower baseline signal than scoring individual probes, better reflecting cardiomyocyte contribution to the mixture (Figure 5C).

Finally, the ddPCR assay was tested on plasma samples. ddPCR revealed a clear signal in the plasma of MI patients and was able to distinguish well between controls and patients. A lower baseline signal was observed in healthy individuals when scoring only dual-labeled probes, indicating increased specificity (Figure 5D). It can be concluded that the ddPCR assay for cardiac cfDNA provides a rapid and simple alternative to sequencing-based assays.

### EXAMPLE 2

### Analyzing hepatic cell methylation signatures

The clinical standard to assess liver damage is serum measurement of alanine aminotransferase (ALT) and aspartate aminotransferase (AST), reflecting hepatocyte injury leading to release of these enzymes into the blood. While in extensive clinical use, these tests
do have important limitations. First, the enzymes are not absolutely hepatocyte-specific. AST is expressed in cardiac and skeletal muscle, kidney, brain, pancreas, lung, leukocytes and erythrocytes, while ALT is primarily present in the liver and kidneys, with low amounts in the heart and skeletal muscle. Second, liver enzymes do not always reflect the full burden of disease and in various hepatic pathologies have been shown to be insufficient. A possible reason for this is that the enzymes could be released from dying as well as reversibly injured cells, and therefore do not indicate the exact nature of damage to the liver, nor the number of injured cells. Third, ALT and AST have long half-lives (approximately 47 and 17 hours, respectively), which limits the ability to monitor rapid changes in liver damage.

Short-lived fragments of DNA released from dying cells are emerging as a valuable biomarker. cfDNA analysis is used as a liquid biopsy to detect DNA derived from fetal cells, cancer cells, or transplanted organs, based on genetic differences between the host and the tissue of interest. Tissue-specific DNA methylation patterns have been used to determine the tissue origins of cfDNA, allowing the identification of cell death in specific tissues even if they carry a genome that is identical to that of the host. In the present example, the application of this approach is described for the detection of cfDNA derived from hepatocytes. Hepatocyte-specific DNA methylation patterns are uncovered which are then used these as specific biomarkers to detect hepatocyte-derived cfDNA in the plasma of healthy individuals and in people with an injured liver. The results support the potential utility of hepatocyte-specific DNA methylation markers for minimally-invasive, specific and sensitive detection and monitoring of hepatocellular death.

### MATERIALS AND METHODS

***Biomarkers:*** Tissue-specific methylation biomarkers were selected after a comparison of publicly available genome-wide DNA methylation datasets generated using Illumina Infinium HumanMethylation450k BeadChip array. The comparison included in addition the methylome of human cholangiocytes, generated locally by sorting dissociated liver tissue.

***Sample Preparation and DNA Processing.*** Blood samples were collected in EDTA-containing plasma-preparation tubes and centrifuged for 10 min in 4 °C at 1,500 × g. The supernatant was transferred to a fresh 15 ml conical tube without disturbing the cellular layer and centrifuged again for 10 min in 4 degrees at 3000 × g. The supernatant was collected and stored in -80 °C.

Cell-free DNA was extracted from 1-4 mL of plasma using the QIAsymphony liquid handling robot (Qiagen) and treated with bisulfite (Zymo Research). DNA concentration was measured using Qbit double-strand molecular probes (Invitrogen). Bisulfite-treated DNA was PCR amplified using primers specific for bisulfite-treated DNA but independent of methylation status at monitored CpG sites.

Primers were bar-coded, allowing the mixing of samples from different individuals when sequencing products. Sequencing was performed on PCR products using MiSeq Reagent Kit v2 (MiSeq, Illuminamethod) or NextSeq 500/550 v2 sequencing reagent kits. Sequenced reads were separated by barcode, aligned to the target sequence, and analyzed using custom scripts written and implemented in Matlab. Reads were quality filtered based on Illumina quality scores. Reads were identified by having at least 80% similarity to target sequences and containing all the expected CpGs in the sequence. CpGs were considered methylated if "CG" was read and were considered unmethylated if "TG" was read. The efficiency of bisulfite conversion was assessed by analyzing the methylation of non-CpG cytosines.

***Statistical Analysis.*** To assess the significance of differences between groups, we used a two-tailed Mann-Whitney test.

***Digital droplet PCR.*** Bisulfite-treated cfDNA was interrogated using methylation-sensitive TaqMan^{™} probes. The limited length of probes (up to 30 bp) dictated that they could cover only 2 to 4 informative CpG sites. In the IGF2R locus 4 CpGs were covered. However, in the VTN= locus, only 2 CpGs were covered by a probe predicting a relatively high frequency of "noise" (positive droplets) in DNA from non-liver tissue. In the sequencing-based assay, this problem was addressed by documenting the methylation status of 5 adjacent cytosines (Figure 10A), which greatly increased specificity. To implement this concept in the ddPCR platform, two Taqman^{™} probes were designed, each recognizing lack of methylation in a different cluster of cytosines (each containing 2 CpG sites) within the same amplified 100bp fragment from the VTN locus. Each probe was labeled with a different fluorophore, such that it was possible to identify droplets in which both probes found a target. Such droplets would be interpreted as containing a VTN cfDNA fragment in which all 4 targeted cytosines were unmethylated. This resulted in a ddPCR assay with the improved specificity afforded by interrogating multiple cytosines on the same DNA molecule.

Each 20 µL volume reaction mix consisted of ddPCR^{™} Supermix for Probes (No dUTP) (Bio-Rad), 900 nM primer, 250 nM probe, and 2 µL of sample. The mixture and droplet generation oil were loaded onto a droplet generator (Bio-Rad). Droplets were transferred to a 96-well PCR plate and sealed. The PCR was run on a thermal cycler as follows: 10 minutes of activation at 95 °C, 47 cycles of a 2 step amplification protocol (30 s at 94°C denaturation and 60 s at 56 °C), and a 10-minute inactivation step at 98 °C. The PCR plate was transferred to a QX100 Droplet Reader (Bio-Rad), and products were analyzed with QuantaSoft (Bio-Rad) analysis software. Discrimination between droplets that contained the target (positives) and those that did not (negatives) was achieved by applying a fluorescence amplitude threshold based on the amplitude of reads from the negative template control.

### Probe and primer sequences:

***VTN:***
   Probe 1 - SEQ ID NO: 178
   Probe 2 -- SEQ ID NO: 179
   Primer 1 - forward - SEQ ID NO: 180
   Primer 2 -- reverse - SEQ ID NO: 181.
***IGF2R:***
   Probe 1 - SEQ ID NO: 182
   Primer 1- forward - SEQ ID NO: 183
   Primer 2 - reverse - SEQ ID NO: 184
***ITIH4**:* Primer 1- forward -SEQ ID NO: 185
***ITIH4:*** Primer 2 reverse - SEQ ID NO: 186

### RESULTS

### Identification of hepatocyte-specific DNA methylation markers

By comparing publicly available human tissue methylomes the present inventors selected three genomic loci, adjacent to the ITIH4, IGF2R and VTN genes, which were unmethylated in the liver compared with other tissues and cell types (Figures 17A-C and Figure 10A). Moreover, the loci were unmethylated in DNA from purified hepatocytes and methylated in purified cholangiocytes, indicating cell type specificity (Figures 17A-C). Experiments with genomic DNA extracted from a panel of human tissues indicated that the sequence of each of these loci includes 4-7 cytosine-guanine dinucleotides (CpG sites), which are fully unmethylated in hepatocytes and methylated in all other tissues (Figure 10B and methods). The human liver DNA was spiked with human leukocyte DNA in different proportions and unmethylated ITIH4, IGF2R and VTN molecules were quantified. Excellent correlation between the measured methylation signal and the input material was found, and a liver signal was detected even when liver DNA was diluted 1:1,000 in leukocyte DNA (Figure 10C). Thus, the present inventors have defined short sequences of DNA, whose methylation status constitutes an epigenetic signature unique to hepatocytes relative to blood cells and other tissues, allowing detection of hepatocyte-derived DNA in mixed samples.

### Hepatocyte-derived cfDNA in healthy individuals

The frequency of unmethylated ITIH4, IGF2R and VTN molecules was measured in plasma, reasoning that the presence of hepatocyte-derived genomic DNA fragments would reflect the rate of hepatocyte death in an individual. cfDNA was prepared from plasma samples, and bisulfite conversion, PCR amplification and massively parallel sequencing was performed, as described in Example 1, to assess how many copies of each marker were present in plasma. Healthy individuals had on average 30 hepatocyte genomes/ml plasma, reflective of basal liver turnover (Figure 11A, B and Figures 18A, B).

### Hepatocyte cfDNA following liver transplantation

To validate the hepatocyte cfDNA assay in a setting where massive hepatocyte death is expected, the present inventors examined plasma samples from 18 patients that underwent liver transplantation. They recorded low levels of hepatocyte cfDNA before transplantation and a very strong signal shortly after reperfusion of the transplant, which declined dramatically in the days that followed (Figure 12A, Figures 19A-C). Hepatocyte cfDNA correlated with the liver enzymes ALT and AST, providing another level of validation to the cfDNA assay (Figure 12B).

The present inventors further examined plasma samples from 6 liver transplant recipients that were sampled periodically after transplantation, including episodes of acute rejection. All six sets of samples showed a similar pattern of hepatocyte cfDNA: low levels before the procedure, and high levels following transplantation, which declined to baseline by day 45 post-transplantation. In 5/6 of the patients the hepatocyte cfDNA signal was elevated during biopsy-proven rejection; strikingly, in 3 patients, elevated hepatocyte cfDNA was recorded prior to clinically detectable rejection (Figure 12C).

***Hepatocyte-derived cfDNA following liver donation:*** Next, the present inventors examined the levels of hepatocyte cfDNA post partial hepatectomy. They examined 14 sets of plasma samples from healthy individuals who donated part of their liver. Patients were sampled prior to partial hepatectomy, and 12, 30 and 95 days after, where liver regeneration should have been complete. They observed abnormally high hepatocyte cfDNA 12 days after surgery, which later declined (Figure 13, Figures 20A-C). Strikingly, by day 12 post surgery the levels of ALT and AST were already back within the normal range, but still above the baseline of each individual. These findings suggest that hepatocyte cfDNA measurement might be a more sensitive indicator of liver damage than enzyme measurements, at least under some circumstances.

### Hepatocyte-derived cfDNA in sepsis patients

Sepsis often involves massive liver damage, in addition to cell death in multiple additional tissues. To examine how liver damage in this complex setting is reflected in hepatocyte-derived cfDNA, the present inventors analyzed 56 plasma samples obtained during sepsis. The amount of hepatocyte-derived cfDNA was significantly higher in sepsis patients compared with healthy controls (Figures 14A-B and Figures 21A-C). Hepatocyte cfDNA values were in good correlation with the levels of liver enzymes ALT and AST measured in the same patients (Figures 14A-B), supporting specificity of hepatocyte cfDNA measurements even in the background on extensive cell death in multiple tissues.

### Hepatocyte-derived cfDNA in Duchenne muscular dystrophy patients

Patients with Duchenne Muscular Dystrophy (DMD) and other muscle dystrophies often present with high levels of ALT and AST, which are derived from damaged muscle rather than from the liver. The present inventors examined whether the hepatocyte-specific methylation markers are elevated in this setting. They tested plasma samples of 10 DMD patients who had elevated levels of ALT and AST. In all samples the concentration of hepatocyte-derived cfDNA was in the normal range (Figure 15), consistent with a non-liver origin of AST and ALT and further supporting specificity of the hepatocyte cfDNA assay.

### A digital droplet PCR assay for measurement of hepatocyte cfDNA

Primers were designed for digital droplet PCR (ddPCR) after bisulfite conversion of cfDNA, and probes were designed that recognize blocks of unmethylated CpGs in the amplified marker regions. VTN and IGF2R markers were used, which had multiple CpGs in close proximity.

ddPCR using both amplicons showed no signal in leukocyte DNA and a strong signal in hepatocyte DNA (Figure 16A). The present inventors then examined 6 sets of plasma samples from 6 patients before and after liver transplantation (the same samples used in Figures 11A-G). Similar to the sequencing results, the ddPCR assay revealed a strong and transient elevation of hepatocyte cfDNA in plasma shortly after transplantation, which declined thereafter, strongly suggesting validity of the assay (Figure 16B).

### EXAMPLE 3

### List of additional identified targets

A list of identified targets is provided in Table 1 and 2 herein below. The targets can be used to identify a cell type of the listed organ. It will be appreciated that the sequences provided are 500 base pairs. Preferably the target sequence which is amplified comprises the nucleotides CG which are at position 250 and 251 of each of these sequences and additional nucleotides up and/or down-stream of this site.

**Table 1**

| ***Organ*** | ***Name*** | ***SEQ ID NO:*** |
|---|---|---|
| Acinar | CPA1 | 2 |
| Acinar | LMF2 | 3 |
| Acinar | NCLN | 4 |
| Acinar | BRF1 | 5 |
| Acinar | FRY | 6 |
| Astrocytes | HDAC4 | 7 |
| Astrocytes | AGAP1 | 8 |
| Astrocytes | AST1 | 9 |
| Astrocytes | PRDM | 10 |
| Astrocytes | FOXP4 | 11 |
| Astrocytes | KIAA | 12 |
| Astrocytes | PRDM2 | 13 |
| Astrocytes | WWOX | 14 |
| B cells | LRP5 | 15 |
| B cells | SORL1 | 16 |
| B cells | TRPV1 | 17 |
| BETA | INSh | 18 |
| BETA | MTG1 | 19 |
| BETA | ZC3H3 | 20 |
| BETA | Leng8 | 21 |
| BETA | Fbxw8 | 22 |
| BETA | Fbxl19 | 23 |
| Blood | Loc1/AGAP2 | 24 |
| Blood | PTPRCAP | 25 |
| BRAIN | MAD1L1 | 26 |
| BRAIN | PTPRN2 | 27 |
| BRAIN | WM1 | 28 |
| BRAIN | MBP | 29 |
| BRAIN | NUMBLE | 30 |
| BRAIN | LRRN3 | 31 |
| BRAIN | cg0978 | 32 |
| BRAIN | ZNF238 | 33 |
| Brain | WB1 | 34 |
| Brain | UBE4B | 35 |
| Breast | KRT19 | 36 |
| Breast | LMX1B | 37 |
| Breast | ZNF296 | 38 |
| CD8 cells | CD8A | 39 |
| CD8 cells | CD8A anti | 40 |
| CD8 cells | CD8B | 41 |
| CD8 cells | CD8B anti | 42 |
| Colon | FGFRL1 | 43 |
| Colon | FAT1 | 44 |
| Colon | col1 | 45 |
| Colon | MG1 | 46 |
| Colon | colnp | 47 |
| Colon | col2np | 48 |
| Colon | ECH1 | 49 |
| Colon | ECH1 | 50 |
| Colon | CNL (mv name) | 51 |
| Colon | MAP7D1 | 52 |
| Colon | col3np (my name) | 53 |
| Eosinophils | PCYT1A | 54 |
| Eosinophils | PCYT1A anti | 55 |
| Heart | FAM101A | 56 |
| Heart | FAM101A AS | 57 |
| kidney | cg00256155 | 58 |
| kidney | PAX2 | 59 |
| kidney | cg15767955 | 60 |
| kidney | MCF2L | 61 |
| kidney | HOXC4 | 62 |
| kidney | PAX2 | 63 |
| Liver | ITIH4 | 64 |
| Liver | SEBOX;VTN | 65 |
| Liver | IGF2R | 66 |
| LUNG | SFTP/A1 | 67 |
| LUNG | SFTP/A2 | 68 |
| LUNG | CLDN18 | 69 |
| LUNG | RAB4 | 70 |
| LUNG | CHST | 71 |
| LUNG | SFTPC | 72 |
| Melanocytes | GALNT3-B | 73 |
| Melanocytes | Melano1 | 74 |
| Melanocytes | Melano1 anti | 75 |
| Melanocytes | RNF207-A | 76 |
| Melanocytes | RNF207-A anti | 77 |
| Melanocytes | RNF207-B | 78 |
| Melanocytes | RNF207-B anti | 79 |
| Monocytes | TCF7L2 | 80 |
| Monocytes | MONO1 | 81 |
| Muscle | MAD1L1 | 82 |
| Muscle | TPO | 83 |
| Muscle | TNNI2 | 84 |
| Muscle | TRIM72;PYDC1 | 85 |
| Neuron | ZNF509 | 86 |
| Neuron | ITFG3 | 87 |
| Neuron | CTBP2 | 88 |
| Neuron | SLC38A10 | 89 |
| neutrophils | DENND3 | 90 |
| neutrophils | NEUT1 | 91 |
| NK | RFC2 | 92 |
| Oligodendrocytes | PLEK | 93 |
| Oligodendrocytes | EVI5L | 94 |
| Oligodendrocytes | ZFP57 | 95 |
| Oligodendrocytes | DNAH | 96 |
| Oral cavity | hH&N1 | 97 |
| Oral cavity | CALML3 | 98 |
| Oral cavity | hH&N4 | 99 |
| Pancreas | CUX2 | 100 |
| Pancreas | PAN4 | 101 |
| Pancreas | REG1A | 102 |
| Pancreas | FRY | 103 |
| Pancreas | BRF1 | 104 |
| Pancreas | PRDM16 (not the same as above) | 105 |
| Pancreatic duct | PRDM16 | 106 |
| Small intestine | ST5 | 107 |
| Small intestine | BANP | 108 |
| Small intestine | SS18L1 | 109 |
| T cells | PRKCH | 110 |
| T cells | SPATA13 | 111 |
| Thyroid | ZNF500 | 112 |
| Thyroid | ATP11A | 113 |
| Treg | FOXP3 | 114 |
| Treg | FOXP3 ANTI | 115 |
| Treg | FOXP3 TSDR | 116 |
| Treg | FOXP3 TSDR anti | 117 |

**Table 2**

| Organ | Name | SEQ ID NO: |
|---|---|---|
| B cells | NAT10 | 121 |
| BETA | GALNTL4 | 122 |
| BETA | cg06081580 | 123 |
| BETA | RGS9 | 124 |
| BETA | DLG5 | 125 |
| BETA | GNAS | 126 |
| BETA | TTC15 | 127 |
| BETA | MAD1L1 | 128 |
| BETA | cg22406334 | 129 |
| BETA | ZDHHC14 | 130 |
| BETA | ZC3H3_a | 131 |
| BETA | SDK1 | 132 |
| BETA | SFRS16 | 133 |
| BETA | PUS3 | 134 |
| BETA | ZC3H3-c | 135 |
| BETA | ACSF3 | 136 |
| BETA | cg19441717 me | 137 |
| White Blood Cells | SNX11 | 138 |
| Cardiomyocytes | Cardio C | 139 |
| Cardiomyocytes | Cardio D | 140 |
| Cardiomyocytes | Cardio E | 141 |
| Cardiomyocytes | Cardio I | 142 |
| Cardiomyocytes | Cardio J | 143 |
| Colon | CNL2 | 144 |
| Colon | CNL | 145 |
| Colon | col3np | 146 |
| Eosinophils | HTT | 147 |
| Eosinophils | ACOT7 | 148 |
| Kidney | ATP11A | 149 |
| Kidney | PAX2-6032 | 150 |
| Kidney | cg00256155 | 151 |
| Kidney | PAX2-818 | 152 |
| Kidney | MCF2L | 153 |
| LUNG | LUAD1 | 154 |
| LUNG | LUAD5 | 155 |
| LUNG | LUSC2 | 156 |
| LUNG | LUSC3 | 157 |
| LUNG | S3-unMe | 158 |
| LUNG | S4-unMe | 159 |
| LUNG | S5-unMe | 160 |
| LUNG | S5-Meth | 161 |
| LUNG | S10-unMe | 162 |
| LUNG | S11-unMe | 163 |
| LUNG | S13-unMe | 164 |
| LUNG | S12-Meth | 165 |
| Melanocytes | RNF207-A | 166 |
| Melanocytes | RNF207-B | 167 |
| Melanocytes | melano1 | 168 |
| Neutrophils | HIPK3 | 169 |
| Oligodendrocyte | NMRAL1 | 170 |
| Oligodendrocyte | TAF8 | 171 |
| Tongue | PIGG | 172 |
| Tongue | MAD1L1 | 173 |
| Tongue | TP73 | 174 |
| Tongue | BAIAP2 | 175 |
| Tongue | HN1L | 176 |
| T regs | FOXP3 TSDR | 177 |

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

### REFERENCES

1. Hickman, P.E. et al. Cardiac troponin may be released by ischemia alone, without necrosis. Clin Chim Acta 411, 318-323 (2010).
2. Michielsen, E.C., Wodzig, W.K. & Van Dieijen-Visser, M.P. Cardiac troponin T release after prolonged strenuous exercise. Sports medicine 38, 425-435 (2008).
3. Roca, E. et al. The Dynamics of Cardiovascular Biomarkers in non-Elite Marathon Runners. J Cardiovasc Transl Res (2017).
4. Katus, H.A., Remppis, A., Scheffold, T., Diederich, K.W. & Kuebler, W. Intracellular compartmentation of cardiac troponin T and its release kinetics in patients with reperfused and nonreperfused myocardial infarction. Am J Cardiol 67, 1360-1367 (1991).
5. Bianchi, D.W. et al. DNA sequencing versus standard prenatal aneuploidy screening. The New England journal of medicine 370, 799-808 (2014).
6. Dawson, S.J. et al. Analysis of circulating tumor DNA to monitor metastatic breast cancer. The New England journal of medicine 368, 1199-1209 (2013).
7. Snyder, T.M., Khush, K.K., Valantine, H.A. & Quake, S.R. Universal noninvasive detection of solid organ transplant rejection. Proc Natl Acad Sci U S A 108, 6229-6234 (2011).
8. De Vlaminck, I. et al. Circulating cell-free DNA enables noninvasive diagnosis of heart transplant rejection. Sci Transl Med 6, 241ra277 (2014).
9. Lehmann-Werman, R. et al. Identification of tissue-specific cell death using methylation patterns of circulating DNA. Proc Natl Acad Sci U S A 113, E1826-1834 (2016).
10. Sun, K. et al. Plasma DNA tissue mapping by genome-wide methylation sequencing for noninvasive prenatal, cancer, and transplantation assessments. Proc Natl Acad Sci U S A 112, E5503-5512 (2015).
11. Guo, S. et al. Identification of methylation haplotype blocks aids in deconvolution of heterogeneous tissue samples and tumor tissue-of-origin mapping from plasma DNA. Nat Genet 49, 635-642 (2017).
12. Roadmap Epigenomics, C. et al. Integrative analysis of 111 reference human epigenomes. Nature 518, 317-330 (2015).
13. Bergmann, O. et al. Dynamics of Cell Generation and Turnover in the Human Heart. Cell 161, 1566-1575 (2015).
14. Turner, A., Tsamitros, M. & Bellomo, R. Myocardial cell injury in septic shock. Crit Care Med 27, 1775-1780 (1999).
15. Sanfilippo, F. et al. Diastolic dysfunction and mortality in septic patients: a systematic review and meta-analysis. Intensive care medicine 41, 1004-1013 (2015).
16. Hochstadt, A., Meroz, Y. & Landesberg, G. Myocardial dysfunction in severe sepsis and septic shock: more questions than answers? J Cardiothorac Vasc Anesth 25, 526-535 (2011).
17. Friden, V. et al. Clearance of cardiac troponin T with and without kidney function. Clin Biochem (2017).
18. Gauthier, V.J., Tyler, L.N. & Mannik, M. Blood clearance kinetics and liver uptake of mononucleosomes in mice. J Immunol 156, 1151-1156 (1996).
19. Rhodes, A., Wort, S.J., Thomas, H., Collinson, P. & Bennett, E.D. Plasma DNA concentration as a predictor of mortality and sepsis in critically ill patients. Critical care 10, R60 (2006).
20. Shave, R. et al. Exercise-induced cardiac troponin elevation: evidence, mechanisms, and implications. J Am Coll Cardiol 56, 169-176 (2010).
21. Lo, Y.M. et al. Rapid clearance of fetal DNA from maternal plasma. Am J Hum Genet 64, 218-224 (1999).
22. Simpson, J.T. et al. Detecting DNA cytosine methylation using nanopore sequencing. Nature methods 14, 407-410 (2017).

## Claims

1. A method of ascertaining the methylation status of a double-stranded, cell-free DNA molecule in a specimen, the method comprising ascertaining the methylation status of at least two methylation sites of the same double-stranded cell-free DNA molecule, wherein said double-stranded, cell-free DNA molecule comprises a nucleotide sequence which comprises no more than 300 base pairs and is comprised in a sequence as set forth in any one of SEQ ID NOs 64-66, thereby ascertaining the methylation status of a double-stranded, cell-free DNA molecule.

2. A method of detecting death of a cell type or tissue in a subject comprising determining whether a double-stranded, cell-free DNA molecule comprised in a specimen of the subject is derived from the cell type or tissue, wherein said determining is effected by ascertaining the methylation status of at least two methylation sites on a continuous nucleotide sequence of the same double-stranded, cell-free DNA molecule, wherein said continuous nucleotide sequence is no longer than 300 base pairs and is comprised in a sequence as set forth in any one of SEQ ID NOs 64-66.

3. A method of determining whether a double-stranded, cell-free DNA molecule is derived from a cell type or tissue of interest in a specimen, the method comprising: ascertaining the methylation status of at least two methylation sites on a continuous nucleotide sequence of the same double-stranded cell-free DNA molecule, wherein said nucleotide sequence comprises no more than 300 base pairs and is comprised in a sequence as set forth in any one of SEQ ID NOs 64-66, wherein a methylation status of each of said at least two methylation sites on said continuous nucleotide sequence of said double-stranded, cell-free DNA molecule characteristic of said cell type or tissue of interest is indicative that the double-stranded, cell-free DNA molecule is derived from the cell type or tissue of interest.

4. The method of any one of claims 1-3, wherein said at least two methylation sites are not more than 300 bp apart on a single strand of the DNA molecule,
optionally wherein said at least two methylation sites are not more than 150 bp apart on a single strand of the DNA molecule, optionally wherein said DNA molecule is no longer than 150 bp;
or wherein said at least two methylation sites comprises at least three methylation sites,
optionally wherein:
(i) said at least three methylation sites are not more than 300 bp apart on a single strand of the DNA molecule, or
(ii) said at least three methylation sites are not more than 150 bp apart on a single strand of the DNA molecule;
or wherein said at least two methylation sites comprises at least four methylation sites,
optionally wherein:
(i) said at least four methylation sites are not more than 300 bp apart on a single strand of the DNA molecule, or
(ii) said at least four methylation sites are not more than 150 bp apart on a single strand of the DNA molecule.

5. The method of any one of claims 1-3, wherein said methylation status is characteristic of a non-diseased cell type or tissue of interest, or wherein said specimen is a blood sample or a fluid sample selected from the group consisting of blood, plasma, sperm, milk, urine, saliva and cerebral spinal fluid.

6. The method of any one of claims 1-3, wherein said ascertaining is effected using at least one methylation-dependent oligonucleotide,
or wherein said ascertaining is effected using at least one methylation-independent oligonucleotide, optionally wherein said ascertaining is effected using at least one methylation-independent oligonucleotide which targets the forward strand of the double-stranded, cell-free DNA molecule and at least one methylation-independent oligonucleotide which targets the reverse strand of the double-stranded, cell-free DNA molecule;
or wherein said ascertaining is effected using:
(i) digital droplet PCR; or
(ii) a multiplex reaction.

7. The method of any one of claims 1-3, wherein said ascertaining is effected by contacting the DNA molecule in the sample with bisulfite to generate single-stranded DNA molecules of which demethylated cytosines of said single-stranded DNA molecules are converted to uracils.

8. The method of claim 7, further comprising contacting said single-stranded DNA with amplification primers under conditions that generate amplified DNA from said single-stranded DNA following said contacting with said bisulfite.

9. The method of claim 8, wherein:
(i) the method further comprises sequencing said amplified DNA;
(ii) said contacting is effected using at least two non-identical labels; or
(iii) said determining is effected using a single label.

10. The method of claim 8, wherein said ascertaining comprises:
(a) contacting said amplified DNA with:
(i) a first probe that hybridizes to said amplified DNA at a site which comprises the first of said at least two methylation sites; and
(ii) a second probe that hybridizes to said amplified DNA at a site which comprises a second of said at least two methylation sites, wherein said first probe and said second probe are labeled with non-identical detectable moieties, wherein said first probe and said second probe comprise a quenching moiety;
wherein said contacting is effected under conditions that separate said quenching moiety from said first probe and said second probe to generate a non-quenched first probe and a non-quenched second probe; and
(b) analyzing the amount of said non-quenched first probe and said non-quenched second probe in at least one specimen fraction of a plurality of specimen fractions, optionally wherein said first probe hybridizes to the forward strand of said amplified DNA and said second probe hybridizes to the reverse strand of said amplified DNA.

11. The method of any one of claims 1-3, wherein the specimen comprises cell-free DNA which is derived from a second cell which is non-identical to said cell type or tissue, optionally further comprising:
(i) analyzing the amount of cell-free DNA derived from said cell type or tissue: amount of cell-free DNA derived from said second cell; or
(ii) analyzing the amount of cell-free DNA derived from said cell type or tissue: total amount of cell-free DNA in the sample.

12. A kit for identifying the source of DNA in a sample comprising oligonucleotides which are capable of detecting the methylation status of at least two methylation sites in a nucleic acid sequence of the same molecule of DNA, or comprising at least two oligonucleotides which are capable of amplifying a DNA molecule, said nucleic acid sequence being no longer than 300 base pairs and comprising at least two methylation sites which are differentially methylated in a first cell of interest with respect to a second cell which is non-identical to said first cell of interest, wherein said nucleic acid sequence is comprised in a sequence as set forth in any one of SEQ ID NOs 64-66.

13. The kit of claim 12, wherein at least one of said oligonucleotides encodes a bar-code sequence and/or is labeled with a detectable moiety;
or wherein the kit further comprises:
(i) at least one agent for sequencing said DNA sequence;
(ii) DNA having said nucleic acid sequence, wherein said DNA is derived from a known cell of interest;
(iii) bisulfite;
(iv) a Taqman polymerase;
(v) a droplet forming oil; or
(vi) a Taqman polymerase and a droplet forming oil;
or wherein the kit comprises oligonucleotides which are capable of detecting the methylation status of at least two methylation sites in a nucleic acid sequence of the same molecule of DNA, wherein said DNA is cell-free DNA.

14. A method of classifying a disease associated with tissue damage, said disease being selected from the group consisting of sepsis, lupus and HIV, the method comprising analyzing cell-free DNA derived from said tissue in a fluid sample of the subject, wherein the amount of said cell-free DNA is indicative of a classification of the sepsis, lupus or HIV.

15. A method of classifying a disease or disorder associated with tissue damage the method comprising analyzing cell-free DNA derived from said tissue in a fluid sample of the subject, wherein said cell-free DNA comprises a sequence which is comprised in any one of SEQ ID NOs 64-66, wherein the amount of said cell-free DNA is indicative of a classification of the disease or disorder.
